# EUROPEAN PATENT APPLICATION

(11) **EP 4 563 149 A1**
(43) Date of publication of application: **04.06.2025**
(21) Application number: 23383234.4
(22) Date of filing: 30.11.2023
(51) Int. Cl.: A61K 31/4995, A61P 35/00

(54) **LURBINECTEDIN, ECUBECTEDIN AND A RELATED COMPOUND FOR USE IN THE TREATMENT OF SKIN CANCERS**

(71) Applicant: Pharma Mar, S.A., 28770 Madrid (ES)
(72) Inventor: AVILES MARIN, Pablo Manuel, 28770 Madrid (ES); GUILLEN NAVARRO, Maria Jose, 28770 Madrid (ES); COIN, Frédéric, 75013 Paris (FR); EGLY, Jean Marc, 75013 Paris (FR)
(74) Representative: Gurney, Steven

(57) **Abstract**

The present invention relates to the therapeutic treatment of skin cancer, particularly melanoma by using lurbinectedin, PM14 (ecubectedin) and Compound IA.

## Description

### FIELD OF THE INVENTION

The present invention relates to the therapeutic treatment of skin cancer, particularly melanoma by using lurbinectedin, PM14 (ecubectedin) and Compound IA.

### BACKGROUND OF THE INVENTION

Skin cancer is one of the most common cancers in Western countries. Melanoma is the most aggressive form of skin cancer, while representing only 1% of skin-cancer case, is the responsible for more than 80% of skin cancer-related deaths due to its high proclivity for metastasis.

The mitogen-activated protein kinases (MAPK) pathway is a key oncogenic signaling system of a relay of kinases that culminate in cell proliferation, differentiation and survival. Part of the mitogen-activated protein kinase (MAPK) pathway, BRAF gene is essential to the regulation of cellular growth, proliferation, and survival. BRAF gene is mutated in -50% of melanomas.

BRAF^{V600E} is the most frequent BRAF mutation in melanoma, comprising 80% of all BRAF mutations, and resulting in constitutive activation of BRAF and downstream activation of MEK and ERK promoting uncontrolled growth. In addition to BRAF mutations, NRAS and NF1 mutations are found in most of the remaining patients (40%). These genomic alterations are considered driver mutations in melanoma development.

Immunotherapy represents the first-line regimen for metastatic or unresectable melanoma with the application of immune checkpoint inhibitors (nivolumab, pembrolizumab, etc). However, patients harboring BRAF mutations, can benefit from BRAF or MEK targeted therapies.

Current BRAF inhibitors (i.e., vemurafenib, dabrafenib, encorafenib) and MEK (downstream protein within BRAF signaling cascade) inhibitors (i.e., trametinib, cobimetinib) have been approved for targeted treatment of unresectable or metastatic BRAF mutated melanoma, since they have shown improved efficiency.

Despite these recent advances, the most crucial phenomenon hindering clinical success of such therapies remains the intrinsic or acquired resistance to targeted therapy.

In fact, a difficulty in eradicating melanoma lies in the aspect of intratumoural heterogeneity driven by cellular phenotypic plasticity. Melanoma comprises diverse phenotypically subpopulations of cancer cells, with different transcriptional and epigenetic-driver profiles and different sensibilities to the actual treatments. Upon environmental stress or drug therapy, melanoma cells can interconvert between at least two main phenotypes: the proliferative/differentiated MITF high phenotype, also referred to as the "melanocyte-like" state, and the invasive/undifferentiated MITF-low phenotype, referred to as the "mesenchymal-like" state.

Moreover, it has been demonstrated that BRAF mutations in melanoma cells have no relation to phenotype-specific gene expression.

Therefore, this phenotype switching represents one of the main obstacles of clinical success and underline the need of alternative compounds which uniformly target different tumour phenotypes governed by divergent transcriptional programs (with transcriptional factors as MITF, SOX10, SOX9, or c-JUN).

### SUMMARY OF THE INVENTION

The present inventors have surprisingly determined that Compound IA, PM14 and lurbinectedin are effective in the treatment of skin cancer, particularly proliferative/differentiated and/or invasive/undifferentiated melanoma, and melanoma resistant to previous treatments with inhibitors of MAP kinase signaling pathway.

Accordingly, a first aspect of the invention provides Compound IA or a pharmaceutically acceptable salt or ester thereof: 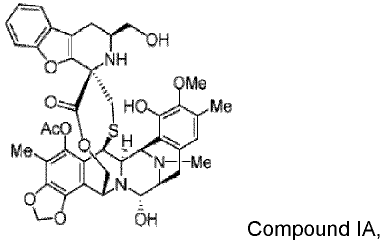 for use in treatment of skin cancer.

In a particular embodiment, the skin cancer is melanoma.

In another particular embodiment, the melanoma is invasive. Melanoma exhibiting the invasive phenotype has previously been hard to treat for which current therapies are not effective.

In another particular embodiment, the melanoma is proliferative/differentiated.

In another particular embodiment, the melanoma is a mixture of proliferative/differentiated and invasive/undifferentiated phenotype. The present invention is effective at treating both phenotypes, for which current therapies are not effective.

In a further aspect, there is provided a method of treatment of skin cancer, the method comprising administering to a patient in need thereof a therapeutically effective amount of Compound IA or a pharmaceutically acceptable salt or ester thereof.

In a further aspect, there is provided the use of Compound IA or a pharmaceutically acceptable salt or ester thereof in the manufacture of a medicament for the treatment of skin cancer, wherein said treatment comprises administering to a patient in need thereof a therapeutically effective amount of Compound IA or a pharmaceutically acceptable salt or ester thereof.

In a further aspect of the present invention, there is provided a pharmaceutical composition or dosage form comprising Compound IA or a pharmaceutically acceptable salt or ester thereof according to the present invention, for use in the treatment of skin cancer.

In a further aspect of the present invention, there is provided the use of a pharmaceutical composition or dosage form comprising Compound IA or a pharmaceutically acceptable salt or ester thereof according to the present invention for the manufacture of a medicament for the treatment of skin cancer.

In a further aspect, there is provided a pharmaceutical package comprising Compound IA or a pharmaceutically acceptable salt or ester thereof, together with instructions for its use in the treatment of skin cancer.

In a further aspect, there is provided a method of inhibiting cancer cell growth, comprising contacting cancer cells with Compound IA or a pharmaceutically acceptable salt or ester thereof, wherein said cancer cells are skin cancer cells.

In a further aspect, the invention provides lurbinectedin or a pharmaceutically acceptable salt or ester thereof: 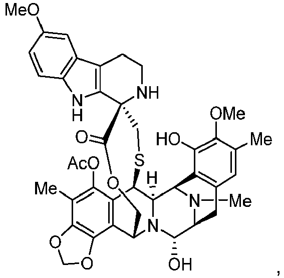 for use in treatment of invasive/undifferentiated and/or proliferative/differentiated melanoma.

In an embodiment, the melanoma is invasive. Melanoma exhibiting the invasive phenotype has previously been hard to treat for which current therapies are not effective.

In a further embodiment, the melanoma is proliferative/differentiated.

In a further embodiment, the melanoma is a mixture of proliferative/differentiated and invasive/undifferentiated phenotype. The present invention is effective at treating both phenotypes, for which current therapies are not effective.

In a further aspect, there is provided a method of treatment of invasive/undifferentiated and/or proliferative/differentiated melanoma, the method comprising administering to a patient in need thereof a therapeutically effective amount of lurbinectedin or a pharmaceutically acceptable salt or ester thereof.

In a further aspect, there is provided the use of lurbinectedin or a pharmaceutically acceptable salt or ester thereof in the manufacture of a medicament for the treatment of invasive/ undifferentiated and/or proliferative/differentiated melanoma, wherein said treatment comprises administering to a patient in need thereof a therapeutically effective amount of lurbinectedin or a pharmaceutically acceptable salt or ester thereof.

In a further aspect of the present invention, there is provided a pharmaceutical composition or dosage form comprising lurbinectedin or a pharmaceutically acceptable salt or ester thereof according to the present invention, for use in the treatment of invasive/ undifferentiated and/or proliferative/differentiated melanoma.

In a further aspect of the present invention, there is provided the use of a pharmaceutical composition or dosage form comprising lurbinectedin or a pharmaceutically acceptable salt or ester thereof according to the present invention for the manufacture of a medicament for the treatment of invasive/ undifferentiated and/or proliferative/differentiated melanoma.

In a further aspect, there is provided a pharmaceutical package comprising lurbinectedin or a pharmaceutically acceptable salt or ester thereof, together with instructions for its use in the treatment of invasive/ undifferentiated and/or proliferative/differentiated melanoma.

In a further aspect, there is provided a method of inhibiting cancer cell growth, comprising contacting cancer cells with lurbinectedin or a pharmaceutically acceptable salt or ester thereof, wherein said cancer cells are invasive/undifferentiated and/or proliferative/differentiated melanoma cells.

In a further aspect, the invention provides PM14 or a pharmaceutically acceptable salt or ester thereof: 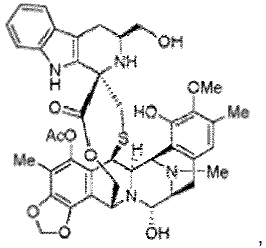 for use in treatment of invasive/undifferentiated and/or proliferative/differentiated melanoma.

In an embodiment, the melanoma is invasive. Melanoma exhibiting the invasive/undifferentiated phenotype has previously been hard to treat for which current therapies are not effective.

In a further embodiment, the melanoma is proliferative/differentiated.

In a further embodiment, the melanoma is a mixture of proliferative/differentiated and invasive/undifferentiated phenotype. The present invention is effective at treating both phenotypes, for which current therapies are not effective.

In a further aspect, there is provided a method of treatment of invasive/ undifferentiated and/or proliferative/differentiated melanoma, the method comprising administering to a patient in need thereof a therapeutically effective amount of PM14 or a pharmaceutically acceptable salt or ester thereof.

In a further aspect, there is provided the use of PM14 or a pharmaceutically acceptable salt or ester thereof in the manufacture of a medicament for the treatment of invasive/ undifferentiated and/or proliferative/differentiated melanoma, wherein said treatment comprises administering to a patient in need thereof a therapeutically effective amount of PM14 or a pharmaceutically acceptable salt or ester thereof.

In a further aspect of the present invention, there is provided a pharmaceutical composition or dosage form comprising PM14 or a pharmaceutically acceptable salt or ester thereof according to the present invention, for use in the treatment of invasive/undifferentiated and/or proliferative/differentiated melanoma.

In a further aspect of the present invention, there is provided the use of a pharmaceutical composition or dosage form comprising PM14 or a pharmaceutically acceptable salt or ester thereof according to the present invention for the manufacture of a medicament for the treatment of invasive/undifferentiated and/or proliferative/differentiated melanoma.

In a further aspect, there is provided a pharmaceutical package comprising PM14 or a pharmaceutically acceptable salt or ester thereof, together with instructions for its use in the treatment of invasive/undifferentiated and/or proliferative/differentiated melanoma.

In a further aspect, there is provided a method of inhibiting cancer cell growth, comprising contacting cancer cells with PM14 or a pharmaceutically acceptable salt or ester thereof, wherein said cancer cells are invasive/undifferentiated and/or proliferative/differentiated melanoma cells.

The following embodiments apply to all aspects of the present invention:
In an embodiment, the melanoma may comprise the invasive/undifferentiated phenotype. The melanoma may comprise at least about 10% invasive/undifferentiated phenotype, at least about 20%, at least about 30%, at least about 40%, at least about 50%, at least about 60%, at least about 70%, at least about 80%, at least about 90%, or around 100% invasive/undifferentiated phenotype.

In an embodiment, the melanoma may comprise the proliferative/differentiated phenotype. The melanoma may comprise at least about 10% proliferative/differentiated phenotype, at least about 20%, at least about 30%, at least about 40%, at least about 50%, at least about 60%, at least about 70%, at least about 80%, at least about 90%, or around 100% proliferative/differentiated phenotype.

In an embodiment, the melanoma may comprise a mixture of proliferative/differentiated and invasive/undifferentiated phenotype.

In an embodiment, the melanoma is unresectable or metastatic BRAF.

In an embodiment, the melanoma is RAS (including NRAS, HRAS and KRAS).

In an embodiment, the melanoma is Triple WT mutated melanoma.

In an embodiment, the melanoma is selected from cutaneous melanoma, mucosal or acral melanoma.

In an embodiment, the melanoma is resistant to previous treatments with inhibitors of MAP kinase signaling pathway.

In an embodiment, the inhibitors of MAP kinase signaling pathway are selected from BRAF or MEK kinase inhibitors or a combination thereof.

In an embodiment, the BRAF or MEK kinase inhibitors are selected from vemurafenib, dabrafenib, encorafenib, trametinib, cobimetinib or binimetinib or a combination thereof.

### BRIEF DESCRIPTION OF THE FIGURES

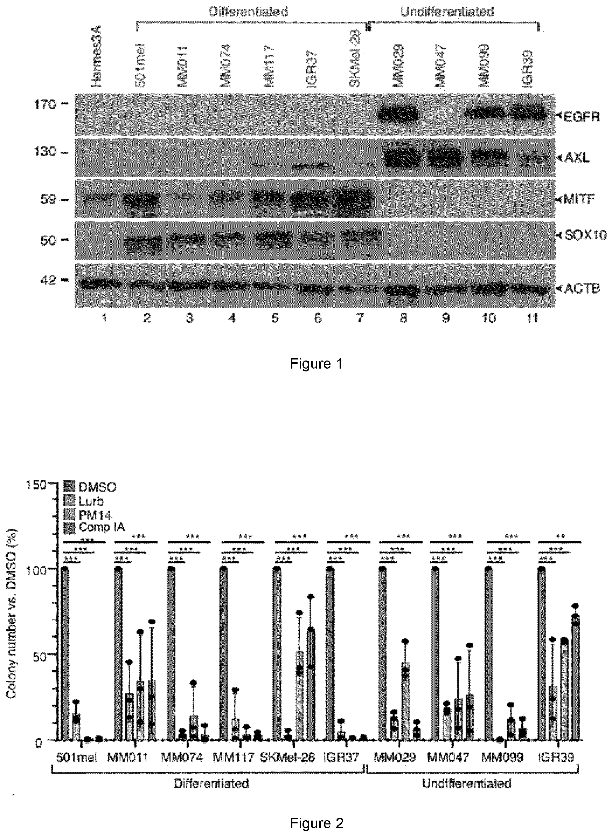

Figure 1 shows protein lysates from either the proliferative/differentiated melanoma cells 501mel, MM011, MM074, MM117, IGR37 and SKMel-28 or the invasive/undifferentiated melanoma cells MM029, MM047, MM099 and IGR39 that were immuno-blotted for proteins. Molecular mass of the proteins is indicated (kDa).
Figure 2 shows melanoma cells treated with DMSO, lurbinectedin, PM14 or Compound IA for48h at respective IC₅₀ concentrations and then allowed to grow for additional 10 days in the absence of the drugs. Results are shown as the mean colony numbers +/- SD for three biological triplicates. P-values are shown (Ordinary one-way ANOVA using Dunnett's multiple comparisons test).

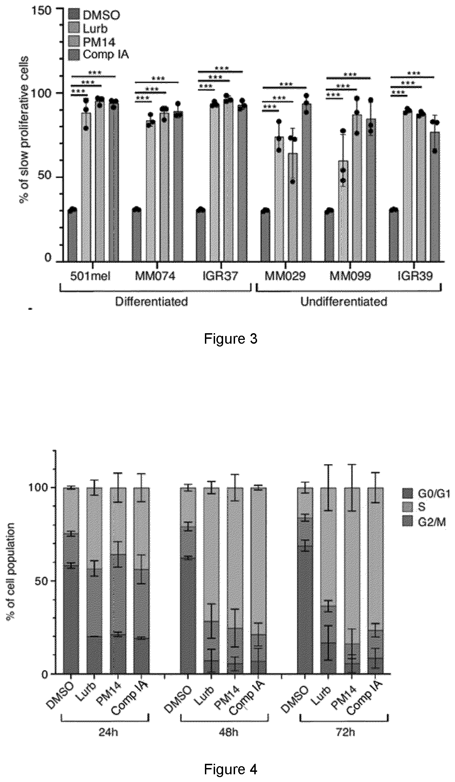

Figure 3 shows melanoma cells that were incubated with CellTrace and subsequently treated with DMSO, lurbinectedin, PM14 or Compound IA for 72h at respective IC₅₀ concentrations. Quantifications of populations with high CellTrace signal in DMSO or drug-treated cells are shown as mean values +/- SD for three biological triplicates. Proliferative cells show low CellTrace signal while non proliferative cells show high CellTrace signal. P-values are shown (Ordinary one-way ANOVA using Dunnett's multiple comparisons test).
Figure 4 shows melanoma cells 501mel treated with DMSO, lurbinectedin, PM14 or Compound IA for 72h at respective IC₅₀ concentrations. Cell cycle was studied by flow cytometry, and results are shown as mean values +/- SD for three biological triplicates.

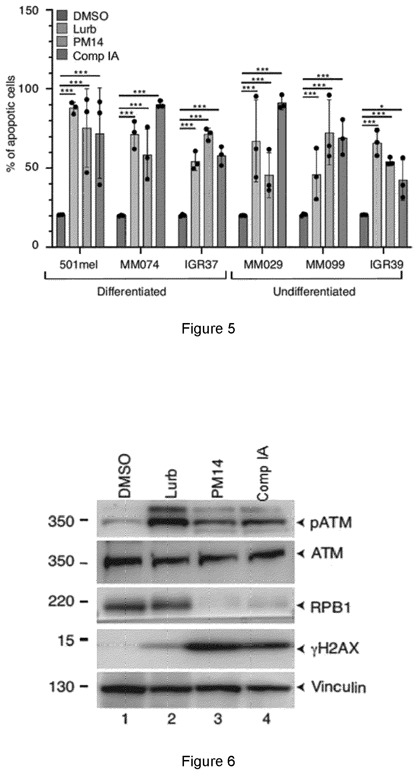

Figure 5 shows melanoma cells treated with DMSO, lurbinectedin, PM14 or Compound IA for 72h at respective IC₅₀ concentrations. Apoptosis was studied by flow cytometry using Annexin V-APC staining. Results are shown as mean values +/- SD for three biological triplicates. P-values are shown (Ordinary one-way ANOVA using Dunnett's multiple comparisons test).
Figure 6 shows protein lysates from the proliferative/differentiated melanoma cell 501mel treated with either lurbinectedin, PM14 or Compound IA for 24h at 5×IC₅₀ concentrations were immuno-blotted for proteins. Molecular mass of the proteins is indicated (kDa).

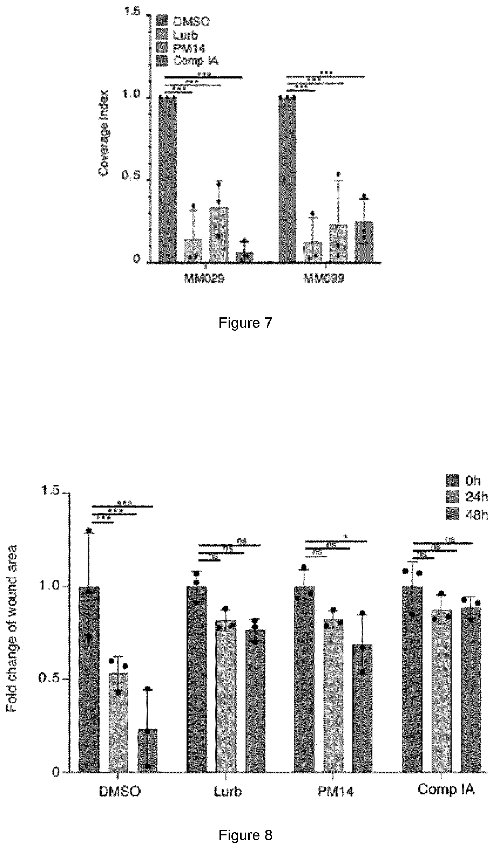

Figure 7 shows MM029 and MM099 melanoma cells treated with DMSO, lurbinectedin, PM14 or Compound IA for 48h at respective IC₅₀ concentrations. Invasion was determined using Boyden chamber assays. Results are shown as mean values of coverage index +/- SD for three biological triplicates. P-values are shown (Ordinary one-way ANOVA using Dunnett's multiple comparisons test).
Figure 8 shows confluent monolayers of melanoma cells 501mel that were scratched and fresh medium containing reduced FCS % and DMSO, lurbinectedin, PM14 or Compound IA (IC₅₀ concentrations) was added. Size of the wound was measured at the indicated times and results are shown as mean values of fold changes of wound area versus DMSO treatment +/- SD for three biological triplicates. P-values are shown (Ordinary one-way ANOVA using Dunnett's multiple comparisons test).

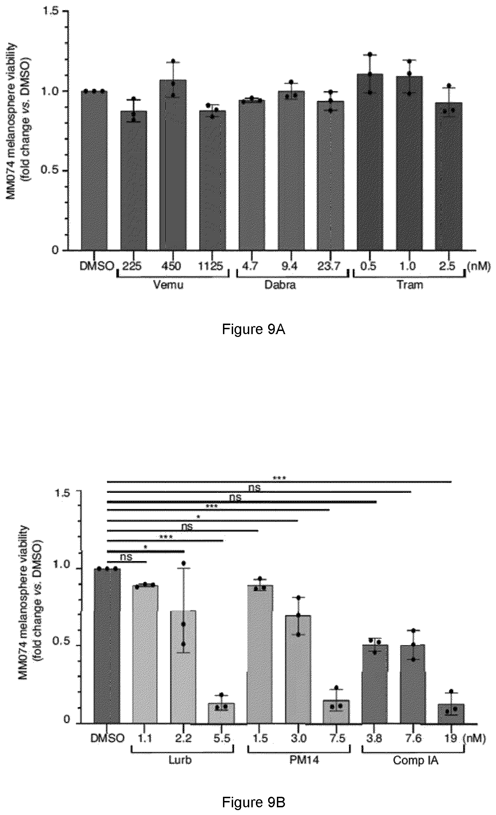

Figures 9A and 9B show MM074 melanospheres treated with trametininb, vemurafenib, or dabrafenib (A) and the compounds of the present invention (B) for 72h, and cell viability was measured with CellTiter-Glo assay, respectively. Results are shown as mean values of viability vs. DMSO +/- SD for three biological triplicates. P-values are shown (Ordinary one-way ANOVA using Dunnett's multiple comparisons test).

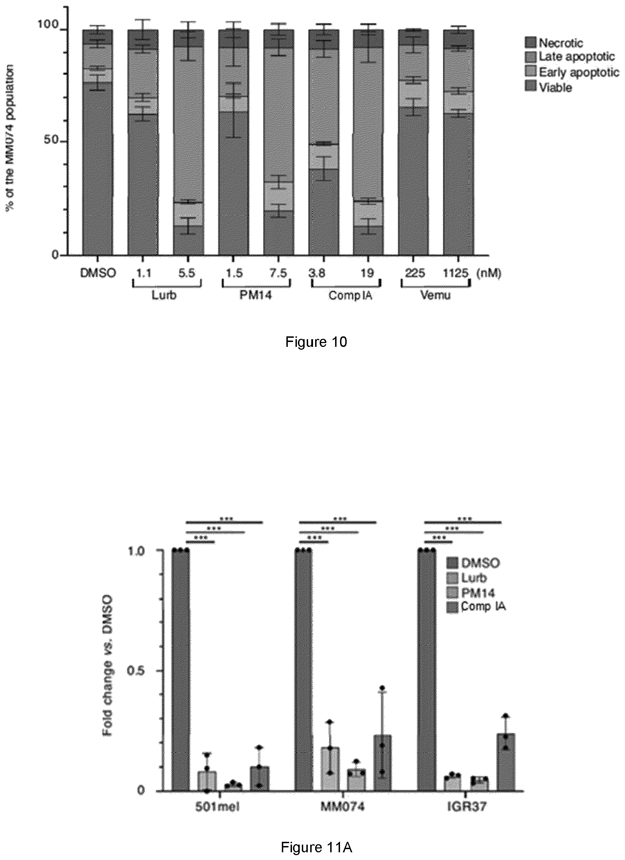

Figure 10 shows MM074 melanospheres treated with DMSO, lurbinectedin, PM14, Compound IA or vemurafenib for 72h. Apoptosis was studied by flow cytometry with Annexin V-APC and propidium iodide staining. Results are shown as mean values +/- SD for three biological triplicates.
Figure 11A shows qRT-PCR analysis showing average 18S-normalized expression of MITF in the differentiated/proliferative MM074, 501mel, and IGR37 cells treated with either vehicle (DMSO), lurbinectedin, PM14 or Compound IA for 12h at 5×IC₅₀ concentrations. Error bars indicate mean values + SD for three biological triplicates. P-values are shown (Ordinary one-way ANOVA using Dunnett's multiple comparisons test).

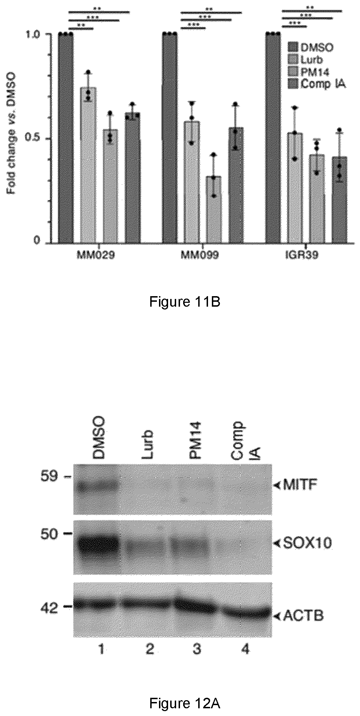

Figure 11B shows qRT-PCR analysis showing average 18S-normalized expression of AXL in the undifferentiated/invasive MM029, MM099, and IGR39 cells treated with either vehicle (DMSO), lurbinectedin, PM14 or Compound IA for 12h at 5×IC₅₀ concentrations. Error bars indicate the mean values +/- SD forthree biological triplicates. P-values are shown (Ordinary one-way ANOVA using Dunnett's multiple comparisons test).

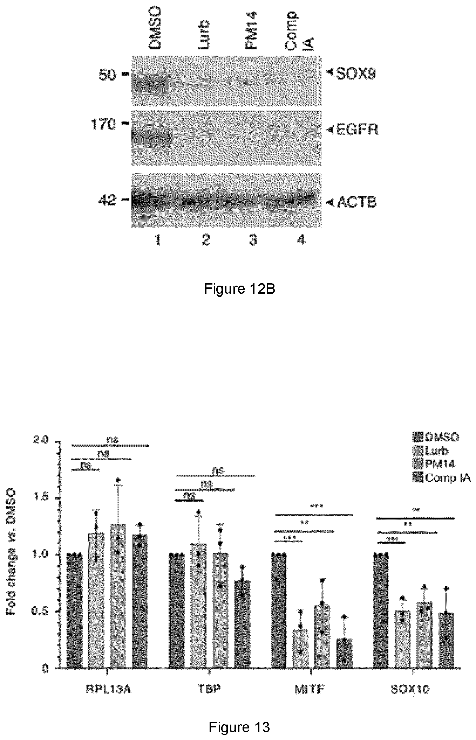

Figures 12A and 12B show the differentiated/proliferative MM074 (A) or undifferentiated/invasive MM099 (B) cells were treated with either vehicle (DMSO), lurbinectedin, PM14 or Compound IA for 24h at 5×IC₅₀ concentrations. Protein lysates were immuno-blotted for proteins as indicated. Molecular mass of the proteins is indicated (kDa).
Figure 13 shows qRT-PCR analysis showing average 18S-normalized expression of RPL13A, TBP, MITF and SOX10 in the MM074 melanospheres treated with either vehicle (DMSO), lurbinectedin, PM14 or Compound IA for 24h at 5×IC₅₀ concentrations. Results are shown as the mean values +/- SD for three biological triplicates. P-values are shown (Ordinary one-way ANOVA using Dunnett's multiple comparisons test).

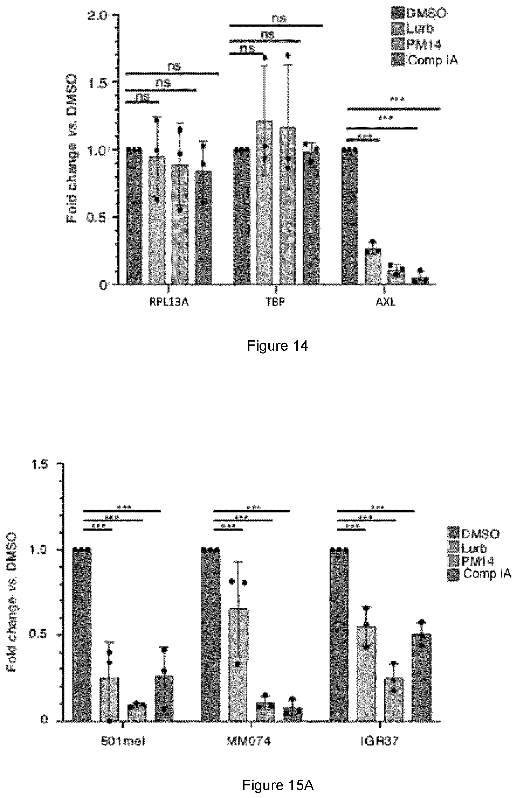

Figure 14 shows qRT-PCR analysis showing average 18S-normalized expression of RPL13A, TBP and AXL in the MM029 melanospheres treated with either vehicle (DMSO), lurbinectedin, PM14 or Compound IA for 24h at 5×IC₅₀ concentrations. Results are shown as the mean values +/- SD for three biological triplicates. P-values are shown (Ordinary one-way ANOVA using Dunnett's multiple comparisons test).

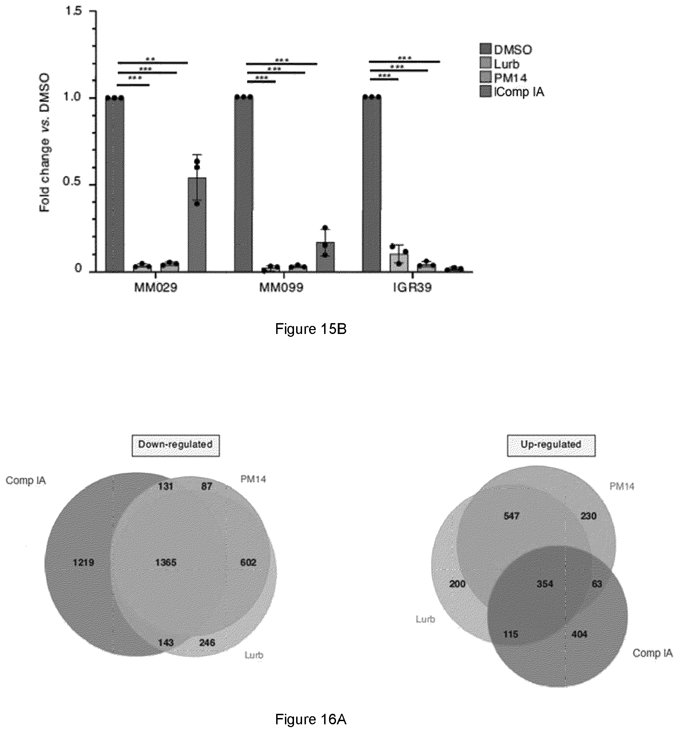

Figure 15 A and 15B show qRT-PCR analysis showing average 18S-normalized expression of SOX10 in indicated differentiated/proliferative cells 501mel, MM074, IGR37 (A) and of EGFR in indicated undifferentiated/invasive cells MM029, MM099, IGR39 (B) treated with either vehicle (DMSO), lurbinectedin, PM14 or Compound IA for 12h at 5×IC₅₀ concentrations. Error bars indicate mean values + SD for three biological triplicates. P-values are shown (Ordinary one-way ANOVA using Dunnett's multiple comparisons test).

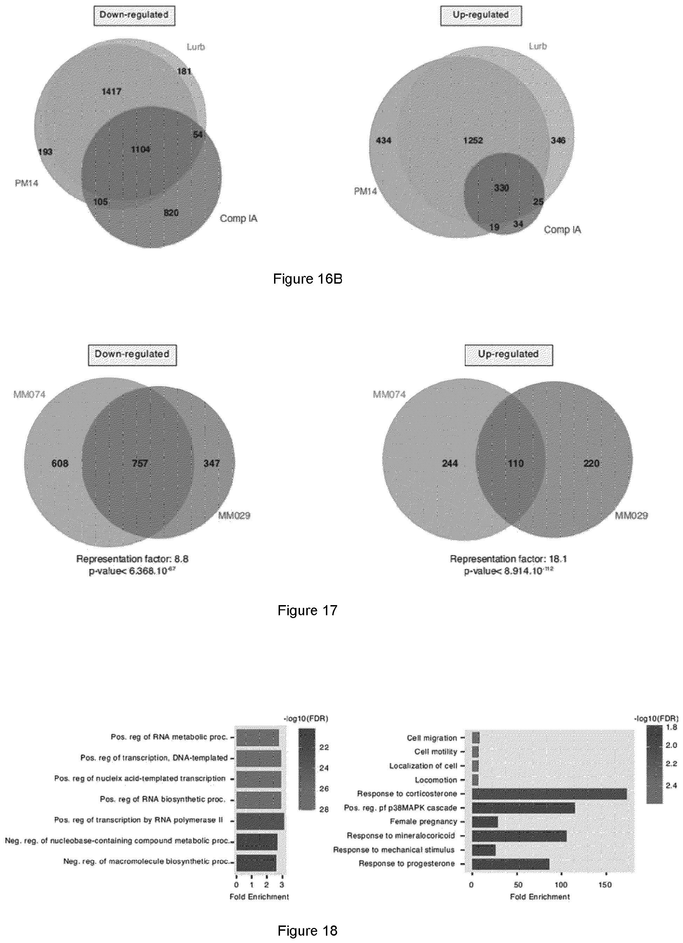

Figures 16A and 16B show Venn diagrams between significantly down-regulated (left) and up-regulated (right) genes identified by RNA-seq in MM074 (A) and MM029 (B) cells upon treatment with either lurbinectedin, PM14 or Compound IA for 8h with 10×IC₅₀ concentrations.
Figure 17 shows Venn diagram between genes identified by RNA-seq as being commonly down-regulated (left) or up-regulated (right) in both MM074 and MM029 cells by the three compounds. Representation factor and hypergeometric p-values are represented.
Figure 18 shows Gene ontology analysis of the 757 genes significantly down-regulated (left) and 110 genes significantly up-regulated in both MM074 and MM029 cells by the three compounds, as identified in (b). The histogram shows the top deregulated biological pathways according to the FDR and fold enrichment.

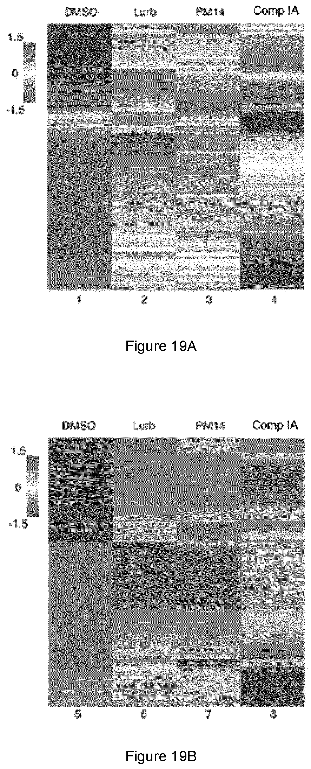

Figures 19A and 19B show heatmaps depicting all deregulated genes from either lurbinectedin, PM14, or Compound IA treatments, in MM074 cells (A) or MM029 cells (B), from the RNA-Seq described in Figures 17A and 17B. RPKM values are represented as z-score.

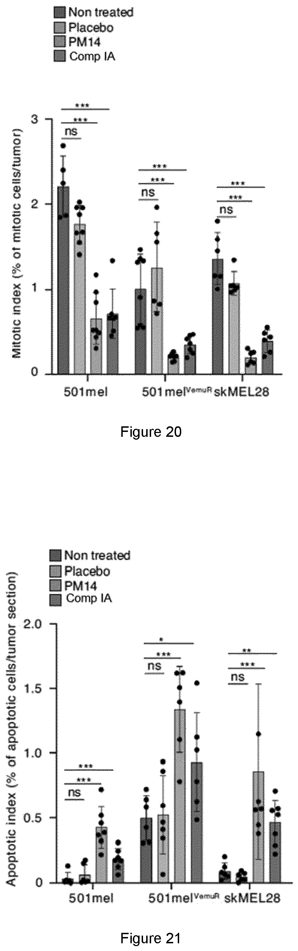

Figure 20 shows quantification of mitotic index (% of pHH3-positive cells/tumour section) of 501mel, 501mel^{Vemur}, and SKMel28 CDX models, after 24h treatment with a one time dose of placebo, PM14 or Compound IA at 1.2 mg/kg, in which results are shown as mean values +/- SD for tumour sections from three tumours per condition. P-values are shown (Ordinary one-way ANOVA using Dunnett's multiple comparisons test).
Figure 21 shows quantification of apoptotic index (% of cleaved caspase-3-positive cells/tumor section) of 501mel, 501mel^{VemuR} and SKMel28 CDX models after 24h treatment with a one-time dose of placebo, PM14 or Compound IA at 1.2 mg/kg, in which results are shown as mean values +/- SD for tumour sections from three tumours per condition. P-values are shown (Ordinary one-way ANOVA using Dunnett's multiple comparisons test).

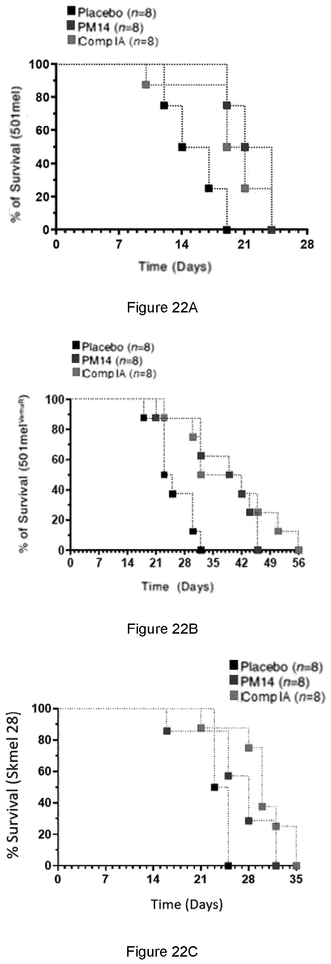

Figures 22A, 22B, and 22C show CDX models (N=8/condition) with 501mel(A), 501mel^{Vemur}(B), and SKmel-28 (C) treated weekly with placebo, PM14 or Compound IA at 1.2 mg/kg and survival was assessed. P-values are shown (log rank (Mantel-Cox) test).

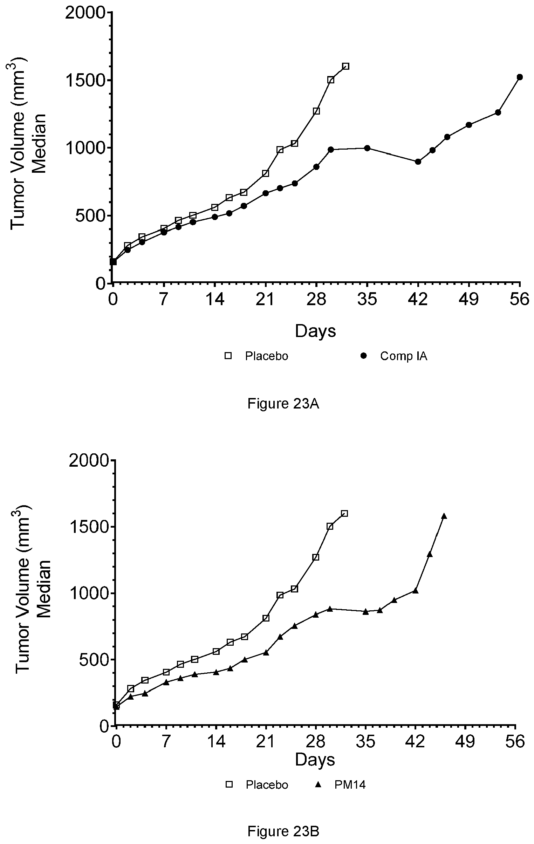

Figures 23A and 23B show tumour volume evaluation on xenografts models with melanoma 501mel^{Vemur} cell line (resistant to Vemurafenib) treated intravenously with Compound IA, and PM14 respectively at 1.2 mg/kg Q7 dx3.

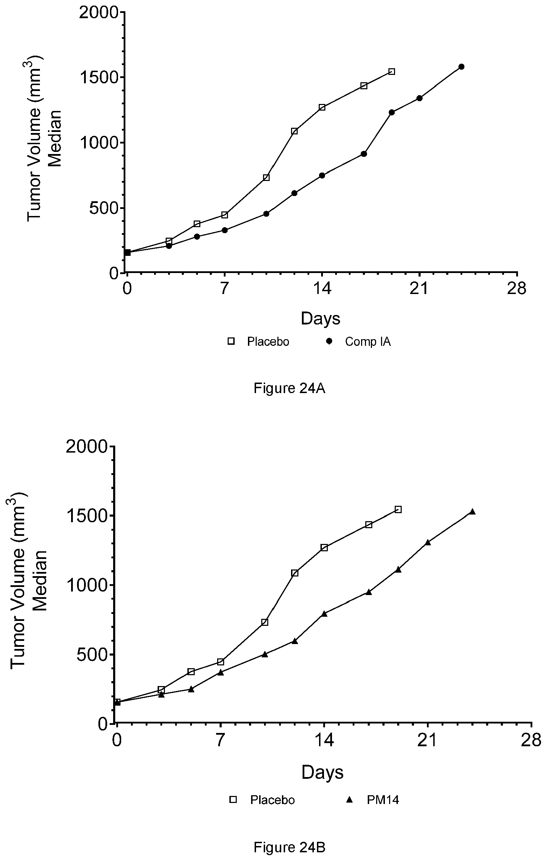

Figures 24A and 24B show tumour volume evaluation on xenograft models with melanoma cell line 501mel treated intravenously with Compound IA, and PM14 respectively at 1.2 mg/kg Q7 dx3.

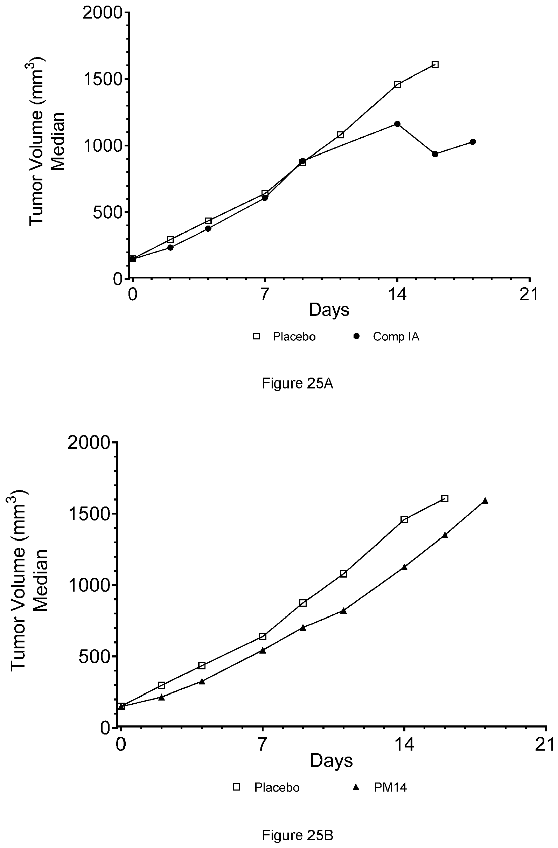

Figures 25A and 25B show tumour volume evaluation on xenograft models with melanoma cell line IGR-37 treated intravenously with Compound IA, and PM14 respectively at 1.2mg/kg Q7 dx3.

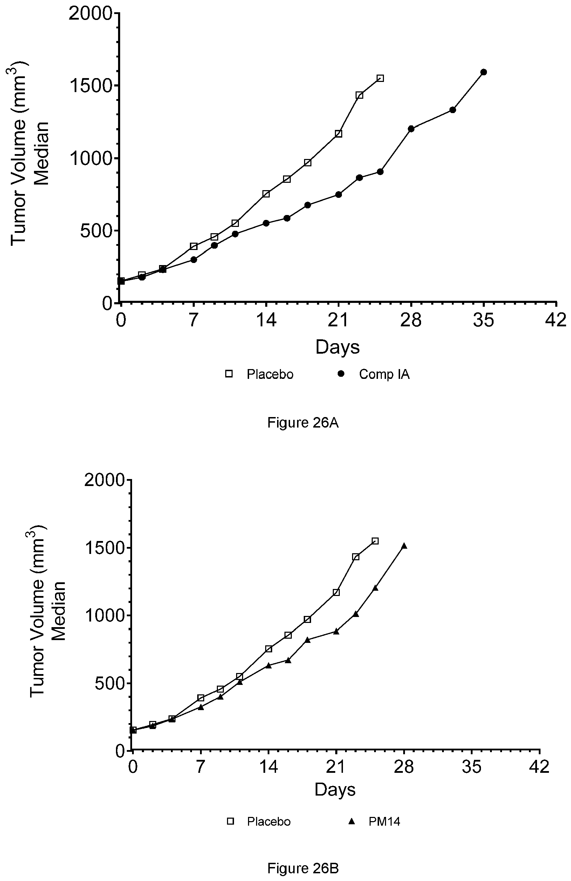

Figures 26A and 26B show tumour volume evaluation on xenograft models with melanoma cell line Skmel-28 treated intravenously with Compound IA, and PM14 respectively at 1.2 mg/kg Q7 dx3.

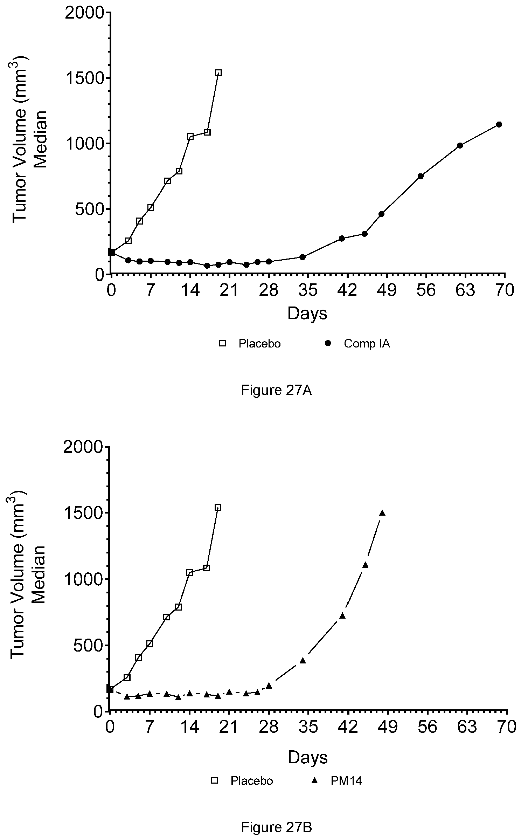

Figures 27A and 27B show tumour volume evaluation on xenograft models with melanoma cell line WM-2664 treated intravenously with Compound IA, and PM14 respectively at 1.2mg/kg Q7 dx3.

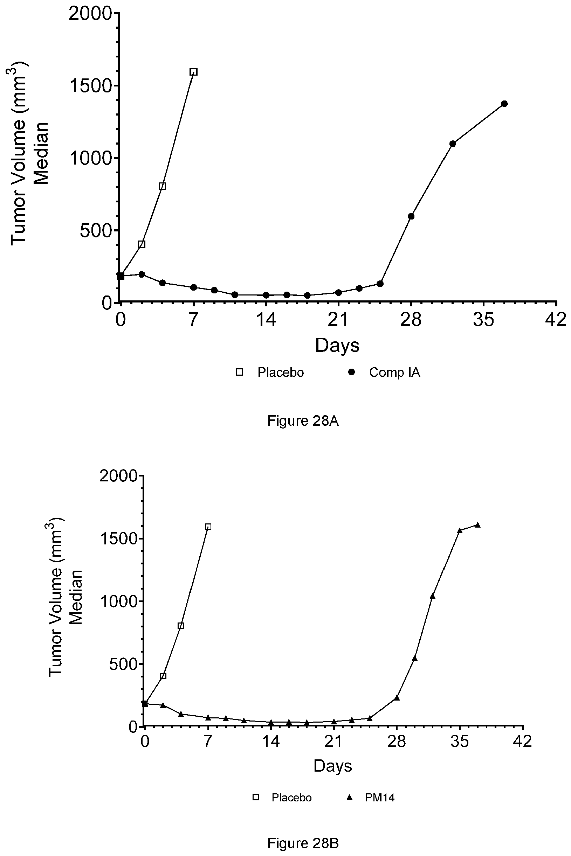

Figures 28A and 28B show tumour volume evaluation on xenograft models with melanoma cell line LOX-IMVI treated intravenously with Compound IA, and PM14 respectively at 1.2 mg/kg Q7 dx3.

### DETAILED DESCRIPTION OF THE INVENTION

In the present application, a number of general terms and phrases are used, which should be interpreted as follows.

The term "treating" as used herein, unless otherwise indicated, means reversing, attenuating, alleviating or inhibiting the progress of the disease or condition to which such term applies, or one or more symptoms of such disorder or condition. The term "treatment" as used herein, unless otherwise indicated, refers to the act of treating as "treating" is defined immediately above.

"Patient" includes humans, non-human mammals (e.g., dogs, cats, rabbits, cattle, horses, sheep, goats, swine, deer, and the like) and non-mammals (e.g., birds, and the like), preferably humans.

The ecteinascidins are exceedingly potent antitumor agents isolated from the marine tunicate *Ecteinascidia turbinata.*

WO2018/197663 describes synthetic ecteinascidin compounds including compound 39-S with the following formula:

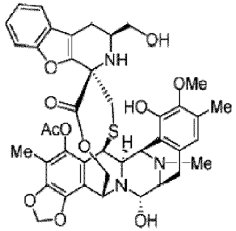

The compound has demonstrated *in vitro* activity against non-small cell lung cancer (NSCLC), colorectal adenocarcinoma, breast adenocarcinoma, pancreas adenocarcinoma, prostate adenocarcinoma, and prostate carcinoma cell lines and *in vivo* activity in fibrosarcoma, breast adenocarcinoma, NSCLC, ovarian carcinoma, gastric carcinoma, small cell lung cancer (SCLC), and prostatic carcinoma xenograft models. This compound can be synthesized using the synthetic route disclosed in WO2018/197663.

WO2018/197663 also describes synthetic ecteinascidin compounds including "ecubectedin", known as PM14, which is described as compound 4-S with the following formula:

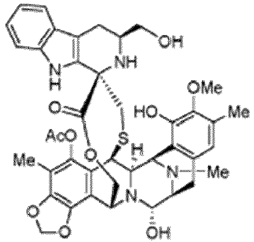

The compound has demonstrated *in vitro* activity against non-small cell lung cancer (NSCLC), colorectal adenocarcinoma, breast adenocarcinoma, pancreas adenocarcinoma, prostate adenocarcinoma, melanoma and prostate carcinoma cell lines and *in vivo* activity in fibrosarcoma, breast adenocarcinoma, NSCLC, ovarian carcinoma, gastric carcinoma, small cell lung cancer (SCLC), prostatic adenocarcinoma, and prostatic carcinoma xenograft models. This compound can be synthesized using the synthetic route disclosed in WO2018/197663.

Lurbinectedin, also known as PM01183 and initially called tryptamicidin, is a synthetic alkaloid with antineoplastic activity, and the subject of WO 03/014127. Lurbinectedin is a selective inhibitor of oncogenic transcription, induces DNA double-strand break generating apoptosis, and modulates the tumour microenvironment. For example, by inhibiting active transcription in tumour-associates macrophages, lurbinectedin downregulates IL-6, IL-8, CCL2, and VEGF. Lurbinectedin can be synthesized using the synthetic route disclosed in WO 03/014127.

The chemical structure of lurbinectedin is represented as follows:

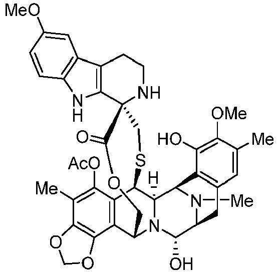

It is a selective inhibitor of the oncogenic transcription programs on which many tumours are particularly dependent. Together with its effect on cancer cells, lurbinectedin inhibits oncogenic transcription in tumour-associated macrophages, downregulating the production of cytokines that are essential for the growth of the tumour. Transcriptional addiction is an acknowledged target in those diseases, many of them lacking other actionable targets.

Lurbinectedin received US approval in 2020 and is marketed in the US for the treatment of adult patients with metastatic small cell lung cancer (SCLC) with disease progression on or after platinum-based chemotherapy. The recommended dosage is 3.2 mg/m2 every 21 days by intravenous infusion. In 2021, lurbinectedin also received marketing authorization in the United Arab Emirates, Canada, Australia and Singapore.

In the context of the present invention, as "skin cancer" it should be understood a cancer that forms in the tissues of the skin. There are several types of skin cancer: melanoma that it is formed in cells in the skin called melanocytes; basal cell carcinoma that forms in the lower part of the epidermis (the outer layer of the skin); squamous cell carcinoma that forms in squamous cells (flat cells that form the surface of the skin); and neuroendocrine carcinoma of the skin that forms in neuroendocrine cells (cells that release hormones in response to signals from the nervous system).

"Melanoma" is a type of skin cancer that develops when melanocytes start to grow out of control. Melanoma is much less common than some other types of skin cancers. However, melanoma is more dangerous because it is much more likely to spread to other parts of the body if not caught and treated early. The number of people diagnosed with melanoma has increased over the last few decades.

Melanomas can develop anywhere on the skin, but they are more likely to start on the trunk (chest and back) in men and on the legs in women. The neck and face are other common sites. Melanomas can also form in other parts of the body, such as the eyes, mouth, genitals, and anal area, but these are much less common than melanoma of the skin. In relation to the present invention, the compounds treat melanoma of the skin and also at any non-skin location.

According to the National Cancer Institute (NCI), 52% of cutaneous melanoma have the BRAF mutant genomic subtype, 28% have the RAS mutant genomic subtype (NRAS, HRAS and KRAS), 14% have the NF1 mutant subtype and 14.5% are Triple WT meaning they are a heterogeneous subgroup lacking BRAF, NRAS, HRAS, and KRAS, and NF1 mutations.

In the context of the invention, "inhibitors of MAP kinase signaling pathway" should be understood as compounds that target components of MAPK pathways.

MAPKs or mitogen-activated protein kinases (MAPKs) are constituents of numerous signal transduction pathways, and are activated by protein kinase cascades. MAPK pathways relay, amplify and integrate signals from a diverse range of stimuli and elicit an appropriate physiological response including cellular proliferation, differentiation, development, inflammatory response and apoptosis in mammalian cells. In the present invention, MAPK pathway is represented by the Raf-MEK-ERK kinase cascade, which produce as cell response proliferation, differentiation and development.

BRAF inhibitors are drugs that can reduce or slow the growth of melanoma in people whose tumors have BRAF mutation. MEK inhibitors are drugs that target kinase enzymes MEK1 and/or MEK2. The MEK gene has a close connection to the BRAF gene, so drugs that are targeted to MEK can also help to treat melanoma having BRAF mutations.

Current BRAF inhibitors include vemurafenib, dabrafenib, encorafenib and MEK inhibitors include trametinib, cobimetinib and binimetinib.

Moreover, positive results led to the US FDA approval of three BRAFi/MEKi combinations, including:
- Dabrafenib plus trametinib (approved in metastatic and resected stage III melanoma, NSCLC, and ATC, and low-grade glioma)
- Vemurafenib plus cobimetinib (approved in metastatic melanoma)
- Encorafenib plus binimetinib (approved in metastatic melanoma)

In an embodiment of the invention, Compound IA is used in melanoma resistant to previous treatments with a MAP kinase signaling pathway inhibitor.

In an embodiment of the invention, PM14 is used in melanoma resistant to previous treatments with a MAP kinase signaling pathway inhibitor.

In an embodiment of the invention, lurbinectedin is used in melanoma resistant to previous treatments with a MAP kinase signaling pathway inhibitor.

In an embodiment, Compound IA is used in a method of treatment for a patient who has progressed from treatment with (or is otherwise determined to be resistant to) MAP kinase signaling pathway inhibitor(s).

In another embodiment, PM14 is used in a method of treatment for a patient who has progressed from treatment with (or is otherwise determined to be resistant to) MAP kinase signaling pathway inhibitor(s).

In a further embodiment, lurbinectedin is used in a method of treatment for a patient who has progressed from treatment with (or is otherwise determined to be resistant to) MAP kinase signaling pathway inhibitor(s).

The MAP kinase signaling pathway inhibitor may be a BRAF inhibitor.

The BRAF inhibitor may be vemurafenib.

The BRAF inhibitor may be dabrafenib.

The BRAF inhibitor may be encorafenib.

The MAP kinase signaling pathway inhibitor may be a MEK inhibitor.

The MEK inhibitor may be trametinib.

The MEK inhibitor may be cobimetinib.

The MEK inhibitor may be binimetinib.

The MAP kinase signaling pathway inhibitor may be both a BRAF inhibitor and MEK inhibitor.

The BRAF/MEK inhibitors may be dabrafenib plus trametinib.

The BRAF/MEK inhibitors may be vemurafenib plus cobimetinib.

The BRAF/MEK inhibitors may be encorafenib plus binimetinib.

The MAP kinase signaling pathway inhibitor may be a MEK inhibitor.

Transcription is deregulated in melanoma. An example of a melanoma-driving transcription factor is MITF, which was identified as a lineage-specific oncogene using an integrative approach in which single nucleotide polymorphism (SNP) array data were combined with gene expression analysis of the NCI-60 panel of cell lines.

Moreover, transcription factors represent a class of highly dysregulated cellular effectors in BRAF V600E-carrying melanoma cells resistant to BRAF inhibition.

Compound IA, PM14 or lurbinectedin can balance the dysregulation produced by transcriptional factors. Compound IA, PM14 or lurbinectedin induce a damage response and a significant degradation of the largest subunit of RNAPII which results in the inhibition of the expression of genes essential for melanoma cells such as MITF or SOX10.

Examples of the administration form include without limitation oral, topical, parenteral, sublingual, rectal, vaginal, ocular and intranasal. Parenteral administration includes subcutaneous injections, intravenous, intramuscular, intrasternal injection or infusion techniques. Preferably, the compositions are administered parenterally. Pharmaceutical compositions of the invention can be formulated using methodology well known in the pharmaceutical art to allow a compound according to the present invention to be bioavailable upon administration of the composition to an animal, preferably human. Compositions can take the form of one or more dosage units, where for example, a tablet can be a single dosage unit, and a container of a compound according to the present invention may contain the compound in liquid or in aerosol form and may hold a single or a plurality of dosage units. A composition intended to be administered by parenteral administration can be prepared by combining a compound of the invention with water, or other physiologically suitable diluent, to form a solution or suspension.

### EXAMPLES

### Example 1: Cellular models of human melanoma with distinct genotypes and phenotypes are highly sensitive to lurbinectedin/PM14/Compound IA

To investigate the response of human melanoma cells to lurbinectedin, PM14 or Compound IA, cells representing the two primary phenotypes (invasive or proliferative) and the most prevalent driver mutations in melanoma, as well as human melanocytic cells (generated in vitro in laboratory, Hermes 3A cell line), were examined (Table 1).

**Table 1. Melanoma cell lines used for testing the cytotoxicity of the compounds of the present invention. The phenotype and genotype of these cells is indicated.**

| **Proliferative MITF+/SOX10+AXL-** | **Invasive AXL+/ MITF-/ SOX10-** | **Non-cancerous cells** |
|---|---|---|
| 501 mel (BRAF ^{V600E}) | MM029 (BRAF ^{V600K}) | Hermes 3A |
| MM011 (NRAS ^{061K}) | MM047 (NRAS ^{Q61R}) | |
| MM074 (BRAF ^{V600E}) | MM099 (BRAF ^{V600E}) | |
| MM117 (Triple-WT) (*) | IGR39 (BRAF ^{V600E}) | |
| SKMel-28 (BRAF ^{V600E}) | | |
| IGR37 (BRAF ^{V600E}) | | |

| | | |
|---|---|---|
| (*) Note: Triple negative refers to BRAF, NRAS and NF1 genes | | |

On one hand, differentiated/proliferative patient-derived melanocytic-type cultures MM011 (NRAS^{061K}), MM074 (BRAF^{V600E}), MM117 (Triple-WT) as well as melanoma cell lines 501mel (BRAF^{V600E}), IGR37 (BRAF^{V600E}) and SKMel-28 (BRAF^{V600E}) were evaluated. On the other hand, patient-derived undifferentiated/invasive, mesenchymal-like melanoma cell cultures MM029 (BRAF^{V600K}), MM047 (NRAS^{Q61R}), MM099 (BRAF^{V600E}) and the melanoma cell line IGR39 (BRAF^{V600E}) were examined.

Cells were grown at 37°C in 5% CO₂ (10% for Hermes 3A) and were regularly checked for mycoplasma contamination. MM patient-derived short-term melanoma cultures (MM011, MM074, MM117, MM029, MM047, MM099) were grown in HAM-F10 (Gibco, Invitrogen) supplemented with 10% Fetal Calf Serum (FCS), 25 mM HEPES, 5.2 mM GLUTAMAX and penicillin-streptomycin. Melanoma cell lines 501mel and SKmel28 were grown in RPMI w/o HEPES (Gibco, Invitrogen) supplemented with 10% FCS and gentamycin. Vemurafenib-resistant cells (501melVemuR and MM074VemuR) were additionally supplemented with 1.5 µM of vemurafenib. Melanoma IGR cell lines (IGR37 and IGR39) were grown in RPMI w/o HEPES (Gibco, Invitrogen) supplemented with 15% FCS and gentamycin.

Proliferative/differentiated cells exhibited moderate to high expression of the lineage-specific transcription factors MITF and SOX10, coupled with low to undetectable levels of the prometastatic EGFR and AXL (Table 2 and Figure 1).

Invasive/undifferentiated cells showed low to undetectable levels of MITF and SOX10, along with high levels of EGFR and/or AXL.

To determine the IC₅₀ (half maximal inhibitory concentration) of these cells to various inhibitors, cell viability assays were performed.

After 72h of treatment with different concentrations of the compounds, cells were treated with PrestoBlue reagent according to the manufacturer's instructions. The absorbance per well was measured with a Cellinsight CX5 microplate reader. Data were analyzed using Prism9 statistical software. IC₅₀ values obtained for each cell line are indicated in Table 2.

**Table 2. IC₅₀ obtained in each cell line obtained with the compounds of the present invention in comparison with current used inhibitors vemurafenib (Vemu), dabrafenib Dabra), trametinib (Trame).**

| **Cells** | **Vemu (nM)** | **Dabra (nM)** | **Trame (nM)** | **Lurb (nM)** | **PM14 (nM)** | **Comp IA (nM)** |
|---|---|---|---|---|---|---|
| **Hermes3A** | >10000 | >50 | >50 | 2.67 | 2.89 | 4.93 |
| **501mel** | 2440 | >50 | 3 | 0.37 | 1.25 | 1.74 |
| **MM011** | >10000 | >50 | >50 | 0.47 | 1.07 | 1.44 |
| **MM074** | 225 | 4.68 | 0.51 | 1.06 | 1.15 | 3.81 |
| **MM117** | 488 | >50 | 10.21 | 0.30 | 0.72 | 1.16 |
| **SKMEL-28** | >10000 | 1 | 5.12 | 1.17 | 1.31 | 1.74 |
| **IGR37** | 517 | 25.3 | 0.53 | 0.50 | 1.05 | 1.48 |
| **501mel^{VemuR}** | >10000 | >50 | >50 | 1.07 | 1.04 | 2.13 |
| **MM074^{VemuR}** | >10000 | >50 | 4.65 | 1.36 | 1.63 | 3.08 |
| **MM029** | >10000 | >50 | >50 | 1.28 | 1.40 | 4.73 |
| **MM047** | >10000 | >50 | >50 | 0.45 | 0.96 | 1.38 |
| **MM099** | >10000 | >50 | >50 | 0.73 | 1.41 | 1.85 |
| **IGR37** | >10000 | >50 | >50 | 0.41 | 0.78 | 0.84 |

The patient-derived cell cultures and melanoma cell lines exhibited varying sensitivities to targeted therapy agents commonly used in the clinical management of melanoma, such as the BRAF inhibitors vemurafenib and dabrafenib (Table 2), as well as the MEK inhibitor trametinib (Table 2). Differentiated/proliferative BRAF^{V600E} melanoma cells, such as MM074 or IGR37, were the most responsive to these drugs, while undifferentiated/invasive melanoma cultures and cell lines demonstrated high resistance (micromolar range).

In contrast, it was observed that all melanoma cells displayed high sensitivity to lurbinectedin, PM14 and Compound IA, with IC₅₀ values in the low nanomolar range, spanning from 0.3 to 4.73 nM (Table 2).

Additionally, vemurafenib-resistant cells were generated, namely 501 mel^{Vemur} and MM074^{Vemur}, by exposing cells to increasing drug concentrations *in vitro* (Table 2). These vemurafenib-resistant cells exhibited cross-resistance to dabrafenib (in the case of MM074) and trametinib (Table 2), but remained highly sensitive to lurbinectedin, PM14 and Compound IA (Table 2). When these compounds were tested on the non-cancerous Hermes3A immortalized melanocytes, it was observed that this cell line was less sensitive than the melanoma cells towards lurbinectedin, PM14 or Compound IA.

Collectively, it has been demonstrated that melanoma cells exhibit a high sensitivity to lurbinectedin, PM14 and Compound IA, with IC₅₀ values in the nanomolar ranges, irrespective of the cell phenotypes or driver mutations.

### Example 2: Lurbinectedin/PM14/Compound IA inhibit proliferation, block cell cycle progression, generate DNA damage and lead to melanoma cell apoptotic death

Based on the cell viability assays conducted above, the efficacy of lurbinectedin, PM14 or Compound IA on melanoma cell proliferation was investigated. Initially, a clonogenic assay was performed. Cells were drug-treated at IC₅₀ concentrations during 48h before seeding 1×10³ or 2×10³ cells in 6-well plates without drug, wherein they grew for 10 days to allow for colony formation. Afterwards, cells were fixed for 10min with 4% formaldehyde solution, washed once with PBS and stained with Crystal Violet solution 0.2% for 15 min. The wells were finally washed twice with deionized water, air dried, scanned and analyzed with Fiji software to count the number of colonies.

The results demonstrate a significant impact of lurbinectedin, PM14 and Compound IA on all tested melanoma cell cultures or cell lines (Figure 2).

Subsequently, additional experiments were conducted to elucidate the cellular responses affected in melanoma cells following drug treatments and analyzed proliferation and apoptotic induction using flow cytometry.

Cells (2×10⁶) were seeded in 6 well plates and were incubated 24h later with 1uM of CellTrace Violet reagent (ThermoFisher) according to the manufacturer's instructions, immediately before rinsing and drug treatment at IC₅₀ concentrations. After 48h of incubation, cells were rinsed and incubated with AnnexinV-APC (BD Biosciences). Cell proliferation and apoptosis were detected on a BD LSRFortessaTM Flow Cytometer. Data were analyzed with FlowJo software. To define slow proliferating or apoptotic cells: it was considered that slow proliferating cells represented the 30% of cells with the highest concentration of CellTrace Violet signal in the DMSO control. Then, it was calculated the % of cells that had a signal greater than or equal to this value with drug treatment. For apoptotic cells, it was considered the 20% of cells with the highest signal of AnnexinV-APC in the DMSO control.

For cell cycle analysis, 2×10⁶ cells were seeded in 6 well plates. After drug treatments at IC₅₀ concentrations, cells were pelleted and fixed with 70% ethanol for 1h at 4°C. After 2 washes with cold PBS, cells were incubated with RNAse A and PI for 1h in the dark, before being analyzed on a BD LSRFortessaTM Flow Cytometer. Data were analysed with FlowJo software.

It was observed a significant inhibition of melanoma cell proliferation upon exposure to lurbinectedin, PM14 or Compound IA compared to DMSO (Figure 3). Concurrently, there was a notable blockade of cell cycle progression (Figure 4) and induction of apoptosis (Figure 5) following treatment with lurbinectedin, PM14 or Compound IA.

In order to evaluate possible damage to the DNA, a surrogate biomarker was used: the detection of gamma H2AX (gH2AX), which signals for double strand breaks. Upon treatment with 5×IC₅₀ of each drug for 24h, it was observed distinct gH2AX activation in the nucleus of differentiated/proliferative 501mel melanoma cells or undifferentiated/invasive MM029 cell cultures, which was confirmed by immunoblotting in differentiated/proliferative 501mel and MM074 cells (Figure 6) or undifferentiated/invasive MM029 cells.

In parallel with gH2AX accumulation, phosphorylation of ATM, the master protein involved in the DNA damage response, was observed in 501mel (Figure 6). Interestingly, RPB1 (RNA pol II elongation subunit) degradation was minimal with lurbinectedin, but pronounced in the presence of PM14 and Compound IA (Figure 6).

The results indicate that lurbinectedin, PM14 and Compound IA exert robust cytostatic and cytotoxic effects on both proliferative/differentiated and invasive/undifferentiated melanoma cells, characterized by the generation of DNA breaks and the degradation of RNAPII, particularly notable for PM14 and Compound IA, forcing the cell to apoptotic cell death.

### Example 3: Lurbinectedin/PM14/Compound IA affect the migration and invasion of undifferentiated/invasive melanoma cell cultures

The invasion and migration of undifferentiated/invasive melanoma cell cultures MM029 and MM099 (Figures 7 and 8) due to the presence of lurbinectedin, PM14 or Compound IA was investigated.

Boyden chamber Invasion and wounding-healing assays were performed.

Boyden Chamber Invasion Assay was performed with 2×10⁶ cells that were seeded inside Boyden Chamber inserts (Fisher Scientific) with 4% Matrigel (Corning) and covered with Serum free media. The inserts were placed in 24 well plates filled with complete medium. After 24h, the inserts were fixed for 10 min with 4% Formaldehyde solution, washed once with PBS and stained with Crystal Violet solution 0.2% for 15min. The wells were finally washed twice with deionized water, air dried, and photos were collected using an EVOS xl Core microscope. The pictures were analyzed with Fiji to assess the area of occupancy of the cells.

Wound-healing migration assay was performed with confluent melanoma cell monolayers in 6-well plates that were scratched with the tip of a 20-µL pipette to create uniform, cell-free wounds. Fresh medium with lower FCS % (to mitigate proliferation), with or without drugs, was added. At 0, 24, and 48 hours, photomicrographs of the wounds were taken under an inverted microscope. The wound areas were then quantified using imaged software.

Boyden chamber and wounding assays clearly indicated that all three drugs of the present invention, significantly affected the invasion and migration of undifferentiated/invasive melanoma cell cultures, respectively (Figures 7-8).

### Example 4: Lurbinectedin/PM14/Compound IA exerted strong cytotoxic activities against BRAF-mutant 3D-melanospheres

Using melanosphere culture assays, the effects of lurbinectedin, PM14 or Compound IA on three-dimension (3D) melanoma cultures were investigated. Initially, it was examined the response of 3D melanospheres to BRAF (vemurafenib and dabrafenib) and MEK (trametinib) inhibitors.

MM074 proliferative/differentiated cell line were seeded (5×10⁴ cells) in ultra-low attachment hydrogel-layered 96 well plates in KO DMEM medium supplemented with 20% KSR, AANE, 2 mM Glutamax, Penicillin/Streptomycin and 100 uM Beta-mercaptoethanol. To allow for melanosphere formation, cells were left to grow for 4 days before drug treatment. Then, they were exposed to the compounds of the present invention at [IC₅₀]×1 or [IC₅₀]×5 for 72h and compared with vemurafenib, dabrafenib and trametinib treatment at [IC₅₀]×1 or [IC₅₀]×5 for 72h.

To analyze melanosphere viability after drug treatment, cells were treated with CellTiterGlo reagent (Promega) according to the manufacturer's instructions. Luminescence signals were measured with a Centro XS LB 960 microplate reader (Berthold).

Interestingly, in stark contrast to the response observed in 2D cultures, vemurafenib, dabrafenib and trametinib were unable to reduce cell viability in 3D culture, even at doses equivalent to 5×IC₅₀ (Figure 9A).

Conversely, lurbinectedin, PM14 and Compound IA exhibited significant cytotoxic effects on MM074 melanospheres at doses equivalent to 5×IC₅₀ (Figure 9B).

For apoptosis assays with 3D-grown melanoma cells, TrypLe Select 10x reagent (Gibco) was used to dissociate melanospheres to obtain single-cell suspensions. These cells were incubated with AnnexinV-APC (Biolegend) and Propidium Iodide (PI, Biolegend). With bivariant dot plots, it was distinguished between viable (AnnexinV-/PI-), early apoptotic (AnnexinV+/PI-), late apoptotic (AnnexinV+/PI+) and necrotic cells (AnnexinV-/PI+).

Melanospheres treated with lurbinectedin, PM14 and Compound IA displayed a substantial population of late apoptotic cells positive for Annexin V and propidium iodide (contrary to Vemurafenib), indicating abundant DNA fragmentation compared to control samples (Figure 10).

These findings highlight the potent cytotoxic activity of lurbinectedin, PM14 and Compound IA against mutant-BRAF melanospheres, in contrast to the limited efficacy of currently used targeted therapies based on BRAF and MEK inhibition.

### Example 5: Lurbinectedin, PM14 and Compound IA affect crucial melanoma genes

In order to investigate the mechanisms underlying the sensitivity of melanoma cells to lurbinectedin, PM14 and Compound IA, 2D cell cultures of differentiated/proliferative melanoma cells (501mel, MM074 and IGR37) and undifferentiated/invasive melanoma cells (MM029, MM099 and IGR39) were treated with doses equivalent to 5×IC₅₀ of lurbinectedin, PM14 and Compound IA for 12h.

The aim was to evaluate if lurbinectedin, PM14 and Compound IA deregulate critical genes in melanoma such as lineage-specific transcription factors MITF and SOX10, which are expressed in proliferative/differentiated melanoma cells, and the pro-invasion/migration transmembrane protein AXL and the prometastatic protein EGFR expressed in invasive/differentiated melanoma cells.

RT-qPCR analysis was performed. Total RNA isolation was performed according to the manufacture protocol with NucleoSpin RNA Plus kit (Macherey-Nagel). RNA was retrotranscribed with Reverse Transcriptase Superscript IV (Invitrogen), qPCR was performed with SYBR Green (Roche) and on a LightCycler 480 (Roche). Target gene expression was normalized using 18S as reference gene. Primers for RT-qPCR were designed using Primer-BLAST. The following primers were used:

| | |
|---|---|
| MITF | F CATTGTTATGCTGGAAATGCTAGAA (SEQ ID NO: 1) |
| | R GGCTTGCTGTATGTGGTACTTGG (SEQ ID NO:2) |
| AXL | F CCGTGGACCTACTCTGGCT (SEQ ID NO:3) |
| | R CCTTGGCGTTATGGGCTTC (SEQ ID NO:4) |
| ACTb | F ACATCTGCTGGAAGGTGGAC (SEQ ID NO:5) |
| | R CCCAGCACAATGAAGATCAA (SEQ ID NO: 6) |
| RPL13a | F TTGAGGACCTCTGTGTATTTGTCAA (SEQ ID NO:7) |
| | R CCTGGAGGAGAAGAGGAAAGAGA (SEQ ID NO: 8) |
| TBP | F CGGCTGTTTAACTTCGCTTC (SEQ ID NO: 9) |
| | R CACACGCCAAGAAACAGTGA (SEQ ID NO: 10) |
| SOX10 | F CCAGTTTGACTACTCTGACCATCAG (SEQ ID NO: 11) |
| | R ATATAGGAGAAGGCCGAGTAGAGG (SEQ ID NO: 12) |
| EGFR | F GCAGCGATGCGACCCTC (SEQ ID NO: 13) |
| | R CCAACTGCGTGAGCTTGTTAC (SEQ ID NO: 14) |
| 18S | F TCAACTTTCGATGGTAGTCGCCGT (SEQ ID NO: 15) |
| | R TCCTTGGATGTGGTAGCCGTTTCT (SEQ ID NO: 16) |

RT-qPCR analysis revealed that the expression of MITF and SOX10 in differentiated cells, as well as AXL and EGFR expression in undifferentiated cells, was significantly affected by the compounds of the present invention compared to the housekeeping gene beta-actin, which exhibited stable expression following treatments (Figures 11 A-B and Figures 15 A-B). Furthermore, immunoblotting with antibodies that specifically recognized each protein, was performed 24h after treatment.

Cells were washed once with cold PBS, rinsed with a cell scraper, pelleted and resuspended in LSDB 0.5M buffer (0.5M KCI, 50mM Tris HCl pH 7.9, 20% Glycerol, 1% NP40, 1mM DTT, PIC). Afterwards cells were fully disrupted with 3 cycles of heat shock in liquid nitrogen and 37°C water bath and centrifugated 15min at 14,000rpm to pellet cell debris. Ten micrograms of extract were loaded on SDS-PAGE before transfer to nitro-cellulose membranes and immunoblotting.

Immunoblotting further confirmed a significant decrease in the protein levels of these genes (Figures 12 A-B).

Additionally, in 3D melanoma cultures, the expression of MITF and SOX10 was assessed in MM074 melanospheres and the expression of AXL in MM029 melanospheres, in order to evaluated if it was significantly affected by the compounds of the present invention, when treated with a dose equivalent to 5×IC₅₀ for 24h, as compared to control genes RPL13A or TBP (Figure 13 and Figure 14).

Collectively, these findings indicate that short-term treatments with lurbinectedin, PM14 or Compound IA impact crucial melanoma master regulator genes (MRGs) in both 2D and 3D melanoma cell cultures, leading to cell apoptosis.

### Example 6: Lurbinectedin, PM14 and Compound IA repress differential set of melanoma genes

To investigate the effect of lurbinectedin, PM14 or Compound IA on the transcriptional landscape of melanoma cells, gene expression profiling in 2D cultures of representative melanoma cells, MM074 and MM029, which represent differentiated/proliferative and undifferentiated/invasive phenotypes, was conducted respectively.

The cells were treated with 10×IC₅₀ of the drugs for a short duration of 8h, and total RNA was extracted using QIAquick RNA purification kit (Qiagen). Superscript II RT reverse RT reverse transcription kit (Thermo scientific), according to manufacturer's recommendation. Purified RNA was subjected to library preparation and high throughput sequencing on Illumina NovaSeq as signal-read 50 base reads following manufacturer's instructions. Sequenced samples were analyzed using nf-core RNAseq pipeline v1.3, quality controlled, trimmed, and aligned to the reference human genome using HISAT2. The identification and quantification of transcripts were performed using feature Counts from subread package. Differential expressed gene (DEG) analysis were done using DeSeq2.

Treatment with the compounds of the present invention resulted in a significant down-regulation of genes expressed in both MM074 and MM029 cells, with a smaller number of genes being up-regulated.

Notably, the compounds of the present invention commonly down-regulated 1,365 genes in differentiated MM074 and 1,104 genes in undifferentiated MM029 cells (Figures 16 A-B). Among these genes, 757 were consistently down-regulated by all three drugs in both MM074 and MM029 cells (Figure 17).

Gene ontology (GO; https://geneontology.org/) analysis revealed that many of these 757 genes were involved in transcriptional processes, indicating that a significant fraction of the genes sensitive to lurbinectedin and its derivatives are associated with transcription factor function (Figure 18).

Furthermore, gene set enrichment analysis (GSEA; https://www.gsea-msigdb.org/gsea/index.jsp) demonstrated that genes involved in the G2M checkpoint and WNT beta catenin signaling pathway were particularly affected by the three drugs in both MM074 and MM029.

The transcriptional effects of Compound IA were compared to those of lurbinectedin in differentiated/proliferative MM074 and undifferentiated/invasive MM029 melanoma cell cultures. Compound IA induced distinct transcriptional effects compared to lurbinectedin, with dissimilar and fewer genes being deregulated in both differentiated/proliferative and undifferentiated/invasive melanoma cells (Figures 19 A-B).

Taken together, this demonstrate that lurbinectedin, PM14 and Compound IA have significant impacts on the transcriptional programs of melanoma cells, with Compound IA exerting the lowest influence on transcriptional activity while displaying equally high cytotoxic activity compared to lurbinectedin and PM14.

GSEA and GO analysis further revealed that Compound IA affects a greater number of genes involved in transcriptional regulation, whereas lurbinectedin primarily down-regulates genes associated with the cell cycle pathway.

### Example 7: Anti-proliferative and pro-apoptotic impact of PM14 and Compound IA in xenograft mouse melanoma models

To investigate the effects of the aforementioned tested *drugs in living organisms,* it was decided to examine the impact of PM14 and Compound IA on melanoma cell-derived xenograft (CDXs) mouse models. Human 501mel, 501mel^{VemuR} or SKMEL28 melanoma cells were subcutaneously implanted into the right flank of NSG mice.

Once the tumors reached a size of 150 mm³, a single intravenous (IV) dose of either PM14 or Compound IA at a concentration of 1.2 mg/kg was administrated to the animals (N=3/group).

Large 8-Bits digital scanned images (Hamamatsu, Nanozoomer HT 2.0) of the tumors stained for nuclei (DAPI staining, 10,000 to 30,000 nuclei per section) and pHH3 or Caspase3 were proceeded through an in-house python (v3.8) algorithm to quantify positive cells. Basically, blue channel was proposed to Cellpose2 model (deep learning model backboned by pytorch process) to segment nuclei. Subsequently, nuclei were analyzed for specific signal. For pHH3, nucleus was considered positive if total of pixel above 50 in intensity value exceeds 20% of nuclei surface (in 8 Bits image values range from 0 [no signal] to 255). The same procedure was applied to Caspase3 with pixel value set to 50 and minimal covered surface set to 30%. For each image ratio of positive cells/total nuclei was returned as the experimental variable. Statistics were produced using python's pingouin library (v0.5.3) and Two-way ANOVA and post hoc tests built in functions.

Twenty-four hours later, it was evaluated the level of phosphohistone H3 (pHH3) to measure the mitotic index (% of pHH3-positive cells/tumour section). According to the results, the fraction of pHH3-positive cells decreased fourfold in the 501 mel, 501mel^{VemuR} and SKMEL28-derived xenografts, 24h post-drug treatment, compared to xenografts treated with either no drug or DMSO (Figure 20).

Additionally, it was evaluated the density of apoptotic cells 24 hours (% of cleaved caspase-3-positive cells/tumour section) in 501mel, 501mel^{VemuR} and SKMEL28-derived xenografts, after drug-treatment using caspase-3 cleavage detection through IF. A significant increase in the fraction of apoptotic cells was observed in all three tumours treated with either PM14 or Compound IA, compared to the non-treated or DMSO-treated xenografts (Figure 21).

Tumor volumes were monitored following weekly IV treatments of PM14 or Comp IA at a concentration of 1.2mg/kg. Treatments commenced (Day 0) when the tumors reached 150 mm³ in female NSG mice aged 4 to 6 weeks (N=8/group). We observed statistically significant difference in the antitumor activity of both PM14 and Comp IA, including against the 501mel^{VemuR} -derived tumors, when comparing the groups receiving weekly treatment with the placebo-treated group. Moreover, it was noted a significant increase in overall survival for both drugs (PM14 and Compound IA) in three of the CDX models (Figures 22 A-C), with a particularly pronounced impact on the survival of mice harboring the 501mel^{Vemur}derived tumours (Figure 22B). These findings indicate potent anti-tumor activity of PM14 and Comp IA in living organisms, with significant effect on animal survival.

### Example 8: In vivo tumour volume reduction on xenograft models

Female athymic *nu*/*nu* mice (Harlan Laboratories Models, S.L. Barcelona, Spain or Envigo, Spain) were utilized for all experiments. Animal were housed in individually ventilated cages Sealsafe^{®} Plus, Techmplast S.P.A.), up to ten per cage on a 12-hour light-dark cycle at 21-23 °C and 40-60 % humidity. Mice were allowed free access to irradiated standard rodent diet (Tecklad 2914C) and sterilized water. Animals were acclimated for at least 5 days prior to tumor implantation with a tumor cell suspension.

The cell line used are:
- 501mel
- IGR-37
- SK-Mel-28
- WM-266-4
- LOX-IMVI
- 501mel^{Vemur}

In xenograft studies with cell lines:
- Tumour volume was calculated using the equation (a-b²)/2, where a: length (longest diameter) and b: width (shortest diameter) were measured in mm by using digital caliper (Fowler Sylvac, S235PAT). Tumour dimensions and body weights were recorded 2-3 times per week starting from the first day of treatment.
- When tumors reached ca. 150-250 mm³, tumour bearing animals (N = 8-10/group) were randomly allocated into the treatment groups, based on body weight and tumour measurements by using NewLab Oncology Software (version 2.25.06.00).
- Comparison between median tumour volume of treated and placebo groups was used for evaluation of the antitumoural efficacy.
- Animals were euthanized when their tumours reached ca. 1,500 mm³ and/or severe necrosis was seen.

Treatments producing >20 % lethality and/or 20% net body weight loss were considered toxic.

Placebo was provided in the form of lyophilized cake containing 200 mg sucrose + potassium dihydrogen phosphate 13.6 mg + phosphoric acid q.s. pH 3.8-4.1 + potassium hydroxide q.s. pH3.8-4.1 which was reconstituted with water for infusion.

In these experiments, PM14 and Compound IA, as well as placebo, were intravenously administered once per week for 3 consecutive weeks, on days 0, 7 and 14, whenever it was possible.

Table 3 reports the median tumour volume evaluation with melanoma 501 mel^{Vemur} cell line in mice treated with placebo and intravenous compounds of the present invention PM14 and Compound IA (at a dose of 1.2 mg/kg). The results are also showed in Figures 23 A-B.

**Table 3. Tumour volume evaluation in 501melVemur cell line with the compounds of the present invention PM14 and Compound IA and placebo.**

| **Days** | **Median Tumour Volume (mm³)** | | |
|---|---|---|---|
| | **Placebo** | **Compound IA** | **PM14** |
| 0 | 160.8 | 160.3 | 147.9 |
| 2 | 282.3 | 249 | 225.2 |
| 4 | 344.9 | 306.8 | 248.4 |
| 7 | 406.5 | 378.8 | 330.4 |
| 9 | 466.2 | 417.2 | 361 |
| 11 | 503.3 | 453.7 | 389.7 |
| 14 | 561.8 | 490.8 | 407.1 |
| 16 | 632.9 | 517.4 | 436.4 |
| 18 | 674.1 | 572.1 | 502 |
| 21 | 813.2 | 666.9 | 555.5 |
| 23 | 985.6 | 703.5 | 674.2 |
| 25 | 1,032.4 | 739.3 | 757.7 |
| 28 | 1,272 | 861.2 | 839.8 |
| 30 | 1,503.6 | 986.4 | 882.9 |
| 32 | 1,600.4 | | |
| 35 | | 997.4 | 863.3 |
| 37 | | | 871.8 |
| 39 | | | 948.6 |
| 42 | | 900.1 | 1020 |
| 44 | | 982.3 | 1,295.1 |
| 46 | | 1080 | 1,582.2 |
| 49 | | 1,169.4 | |
| 51 | | | |
| 53 | | 1,262.1 | |
| 56 | | 1,521.8 | |

Compared to the control, the compounds of the present invention produced a reduction of the volume tumour in the different xenograft models with cell line resistant to Vemurafenib. Administration of the compounds of the present invention led to a delay in disease progression.

These results are also showed in Tables 4 to 8 and in Figures 24-28 A-B (501 mel, IGR-37, SK-Mel-28, WM-266-4, LOX-IMVI respectively).

**Table 4. Tumour volume evaluation in 501mel melanoma xenografts with PM14 and Compound IA and placebo.**

| **Days** | **Median Tumour Volume (mm³)** | | |
|---|---|---|---|
| | **Placebo** | **Compound IA** | **PM14** |
| 0 | 158.1 | 158.5 | 158.8 |
| 3 | 247 | 208.3 | 215.5 |
| 5 | 376.8 | 279.8 | 252.3 |
| 7 | 446.7 | 328.5 | 372.7 |
| 10 | 734 | 455.2 | 503.6 |
| 12 | 1,087.7 | 614.1 | 599.4 |
| 14 | 1,270.8 | 749.8 | 797.2 |
| 17 | 1,435.9 | 913 | 950 |
| 19 | 1,544.4 | 1,233 | 1,113.5 |
| 21 | | 1,341.4 | 1,309.8 |
| 24 | | 1,581.4 | 1,530.7 |

**Table 5. Tumour volume evaluation in IGR-37 melanoma xenografts with PM14 and Compound IA and placebo.**

| **Days** | **Median Tumour Volume (mm³)** | | |
|---|---|---|---|
| | **Placebo** | **Compound IA** | **PM14** |
| 0 | 153.3 | 150 | 150.4 |
| 2 | 297.2 | 236.5 | 216.6 |
| 4 | 436.3 | 377.5 | 326.3 |
| 7 | 640.8 | 608 | 544.5 |
| 9 | 874.8 | 886.9 | 704.9 |
| 11 | 1,080 | | 821.6 |
| 14 | 1,459.2 | 1,163.9 | 1,127.4 |
| 16 | 1,607.9 | 936.5 | 1,351.7 |
| 18 | | 1,027.6 | 1,594 |

**Table 6. Tumour volume evaluation in Sk-mel-28 melanoma xenografts with PM14 and Compound IA and placebo.**

| **Days** | **Median Tumour Volume (mm³)** | | |
|---|---|---|---|
| | **Placebo** | **Compound IA** | **PM14** |
| 0 | 153.9 | 152.3 | 152.8 |
| 2 | 195.4 | 179.8 | 184.4 |
| 4 | 238.5 | 232.7 | 235.4 |
| 7 | 392.6 | 302 | 326.2 |
| 9 | 457.6 | 397.8 | 401.5 |
| 11 | 551.1 | 476.6 | 510.6 |
| 14 | 755.2 | 551.1 | 632.6 |
| 16 | 855.8 | 585.8 | 671.9 |
| 18 | 970.3 | 676.6 | 822.8 |
| 21 | 1,169.5 | 748.7 | 884.4 |
| 23 | 1,433.8 | 866.2 | 1,012.5 |
| 25 | 1,551.2 | 906.6 | 1,204.2 |
| 28 | | 1,203.1 | 1,516.7 |
| 30 | | | |
| 32 | | 1,331.3 | |
| 35 | | 1,593.1 | |

**Table 7. Tumour volume evaluation in WM-266-4 melanoma xenografts with PM14 and Compound IA and placebo.**

| **Days** | **Median Tumour Volume (mm³)** | | |
|---|---|---|---|
| | **Placebo** | **Compound IA** | **PM14** |
| 0 | 167.9 | 169.5 | 169.5 |
| 3 | 258.4 | 109.8 | 115.1 |
| 5 | 409 | 100 | 118.2 |
| 7 | 512.3 | 104.4 | 136.6 |
| 10 | 714.3 | 97.8 | 133.1 |
| 12 | 789.7 | 89.2 | 109.8 |
| 14 | 1,052.6 | 94.9 | 138.4 |
| 17 | 1,085.4 | 68.8 | 129.6 |
| 19 | 1,539.1 | 75.7 | 119.2 |
| 21 | | 95.2 | 150.4 |
| 24 | | 75.7 | 137.2 |
| 26 | | 96.7 | 146.9 |
| 28 | | 99.4 | 197.6 |
| 34 | | 134.3 | 388.1 |
| 41 | | 275.2 | 727.7 |
| 45 | | 312.1 | 1,111.6 |
| 48 | | 461.7 | 1,503.6 |
| 55 | | 749.7 | |
| 62 | | 985.3 | |
| 69 | | 1,145.3 | |

**Table 8. Tumour volume evaluation in LOX-IMVI melanoma xenografts with PM14 and Compound IA and placebo.**

| **Days** | **Median Tumour Volume (mm³)** | | |
|---|---|---|---|
| | **Placebo** | **Compound IA** | **PM14** |
| 0 | 184.8 | 186.2 | 185 |
| 2 | 405 | 196.3 | 173.7 |
| 4 | 806.4 | 138.7 | 103.2 |
| 7 | 1,593.6 | 106.8 | 73.8 |
| 9 | | 87.8 | 68.5 |
| 11 | | 56 | 51.2 |
| 14 | | 53.5 | 37.5 |
| 16 | | 54.7 | 40.8 |
| 18 | | 51.4 | 35.3 |
| 21 | | 71.2 | 43.5 |
| 23 | | 99.9 | 55.5 |
| 25 | | 132 | 68.5 |
| 28 | | 597.7 | 233.4 |
| 30 | | 1,158.4 | 547.3 |
| 32 | | 1,098.3 | 1,044 |
| 35 | | 968.8 | 1,565 |
| 37 | | 1,375.6 | 1,610.4 |

Compared to the placebo, PM14 and Compound IA produced a reduction of the volume tumor in the different xenografts with various melanoma cell lines. The compounds of the present invention achieved a delay in disease progression in all melanomas.

### Conclusion:

Low nanomolar doses of lurbinectedin, PM14 and Compound IA all decreased proliferation and invasive capacities of melanoma cells, while inducing apoptosis and cell cycle blockage in the S phase. Similarly, the viabilities of 3D-cultured melanospheres were severely affected by treatments with the three drugs. Surprisingly however, even high doses of MAPKi did not affect the viability of melanospheres containing BRAF^{V600E}-mutated melanocytic MM074 cells, which were shown to be highly sensitive to BRAFi and MEKi in 2D-settings. These results thus showcase the importance of assaying novel drugs in different settings, as factors such as hypoxic signaling or drug penetration might severely limit their therapeutic effects. However, these factors did not seem to limit the efficacy of lurbinectedin, PM14 or Compound IA in the 3D model. Moreover, the three compounds rapidly induced gH2AX in melanoma cells, a phenomenon not observed for MAPKi, marked differences were observed between lurbinectedin and PM14 or Compound IA treatments in some cellular models. In 501mel cells for example, RNAPII degradation and gH2AX were much more pronounced when treated with PM14 or Compound IA, pointing to potential differences in efficacy and intracellular pharmacodynamics.

The results also highlight the dual mechanism of action by which lurbinectedin and PM14 and Compound IA exert their cytotoxic effects. Synchronously, with the induction of DNA breaks, as highlighted through the appearance of gH2AX, drug treatments also led to the important disruption of oncogene expression. Importantly, the transcriptional effects of the compounds seemed to exhibit a high degree of specificity for distinctly overexpressed oncogenes depending on the melanoma cell state. As such, while the expression of housekeeping genes such as ACTb, TBP or RPL13a were not affected in 2D or 3D conditions by short-term drug treatments, oncogenically overexpressed and MRGs such as MITF or SOX10 were heavily inhibited specifically in melanocytic cells. In mesenchymal-like cells however, different genes were affected, such as the overexpressed RTKs AXL or EGFR.

Through their unique mechanism of action, they selectively inhibit the distinct transcription programs on which a given cancer cell subpopulation depends on. As non-cancer cells display a less open chromatin conformation and are less 'transcriptionally addicted', they should experience less cytotoxic effects than cancer cells.

Moreover, GSEA and GO analysis revealed that drug treatments elicited a strong stress response with the activation of AP-1 and EMT factors while disrupting β-catenin-signaling, which represent events related to melanoma phenotype switching.

The compounds of the present invention overcome the negative effects of phenotype switch induction because even mesenchymal-like cells were shown to be sensitive to these drugs.

The *in vitro* and *in vivo* data demonstrate that PM14 and Compound IA significantly impact melanocytic-like melanoma cells, even those with acquired resistances to MAPKi.

The present invention has identified that lurbinectedin, PM14 and Compound IA are effective in the treatment of skin cancer, particularly the treatment of melanoma. The compounds are particularly effective at treating mesenchymal-like melanoma (invasive/undifferentiated phenotype). The compounds are also particularly effective at treating melanocyte-like melanoma, even those with acquired resistances to MAPKi. By being effective across these phenotypes, the compounds of the present invention can mitigate or overcome the negative effects of phenotype switch induction. By overcoming this resistance mechanism used by melanoma to overcome prior therapies, the compounds of the present invention are particularly effective in the treatment of melanoma.

The compounds of the present invention may be used in melanoma patients who have become resistant to previous treatments with inhibitors of MAP kinase signaling pathway. Patients may have de novo resistance. Patients may have progressed from prior treatment with inhibitors of MAP kinase signaling pathway. The inhibitors of MAP kinase signaling pathway may be BRAF inhibitors. The inhibitors of MAP kinase signaling pathway may be MEK kinase inhibitors. The inhibitors of MAP kinase signaling pathway may be a combination of BRAF inhibitors and MEK kinase inhibitors.

Overall, the compounds of the present invention are effective in the treatment of skin cancer. In particular the compounds of the present invention are effective in the treatment of melanoma.

### References

Tsao H et al. Management of cutaneous melanoma. N Engl J Med 2004;351:998-1012
Zhang et al., Cell Research, vol.12, p.9-18, 2002
Flaherty KT et al. Combined BRAF and MEK inhibition in melanoma with BRAF V600 mutations. N Engl J Med 2012;367:1694-703
Nazarian, R. et al. 2010. Melanomas acquire resistance toB-RAF(V600E) inhibition by RTK or N-RAS upregulation. Nature 468, 973-U377.
WO2018/197663
WO 2022243482
Santamaria Nunez, G, et al., Lurbinectedin Specifically Triggers the Degradation of Phosphorylated RNA Polymerase II and the Formation of DNA Breaks in Cancer Cells, Molecular Cancer Therapeutics (2016), 15(10), 2399-2412
Verfaillie, A. et al, 2015. Decoding the regulatory landscape of melanoma reveals TEADS as regulators of the invasive cell state. Nature Communications 6.
Garnett, M.J., Marais, R., 2004. Guilty as charged: B-RAF is a human oncogene. Cancer Cell 6, 313-9.
Ribas, A. et al., J., 2013. Phase I trial evaluating concurrent Vemurafenib and Ipilimumab in patients with advanced BRAF-mutant melanoma. European Journal of Cancer 49, S867-S867.
Siegel, R.L.et al., 2018. Cancer Statistics, 2018. Cancer Journal for Clinicians 68, 7-30.
Casper et al., Use of anti-phosphohistone H3 immunohistochemistry to determine mitotic rate in thin melanoma, Am J Dermatopathol, 32 (2010), pp. 650-654
Melanoma treatment (PDQ)-Health Professional Version-NCI (cancer.gov)
NCCN Guidelines cutaneous melanoma version 2/2023
Decoding the regulatory landscape of melanoma reveals TEADS as regulators of the invasive cell state Nature Communications volume 6, Article number: 6683 (2015).
Systematic classification of melanoma cells by phenotype-specific gene expression mapping - Widmer - 2012 - Pigment Cell & Melanoma Research - Wiley Online Library
Najem A et al., Understanding Molecular Mechanisms of Phenotype Switching and Crosstalk with TME to Reveal New Vulnerabilities of Melanoma. Cells. 2022 Mar 29;11(7):1157.
Garraway LA, et al. Integrative genomic analyses identify MITF as a lineage survival oncogene amplified in malignant melanoma. Nature. 2005;436:117-22.
NCI-SEER-Database, 2023
Akbani, R.., 2015. Genomic Classification of Cutaneous Melanoma. Cell 161, 1681-1696.
Anders, S., Huber, W., 2010. Differential expression analysis for sequence count data. Genome Biol. 11, 1-12.
Arozarena, I., Wellbrock, C., 2019. Phenotype plasticity as enabler of melanoma progression and therapy resistance. Nat. Rev. Cancer 19, 377-391.
Atkins, M.B., 2021. The State of Melanoma: Emergent Challenges and Opportunities. Clin. Cancer Res. 27, 2678-2697.
Benboubker, V., Boivin, F., Dalle, S., Caramel, J., 2022. Cancer Cell Phenotype Plasticity as a Driver of Immune Escape in Melanoma. Front. Immunol. 13, 873116.
Benjamini, Y., Hochberg, Y., 1995. Controlling the False Discovery Rate: A Practical and Powerful Approach to Multiple Testing. J. R. Stat. Soc. Ser. B Methodol. 57, 289-300.
Berico, P., Cigrang, 2021. CDK7 and MITF repress a transcription program involved in survival and drug tolerance in melanoma. EMBO Rep.
Bradner, J.E., Hnisz, D., Young, R.A., 2017. Transcriptional Addiction in Cancer. Cell 168, 629-643.
Bushweller, J.H., 2019. Targeting transcription factors in cancer - from undruggable to reality. Nat. Rev. Cancer 19, 611-624.
Carlino, M.S., Larkin, J., Long, G.V., 2021. Immune checkpoint inhibitors in melanoma. The Lancet 398, 1002-1014.
Chauhan, J.S., 2022. The MITF regulatory network in melanoma. Pigment Cell Melanoma Res. 35, 517-533.
Comandante-Lou, N., 2022. AP-1 transcription factor network explains diverse patterns of cellular plasticity in melanoma cells. Cell Rep. 40, 111147.
Costanzo, F., 2022. Promoters of ASCL1- and NEUROD1-dependent genes are specific targets of lurbinectedin in SCLC cells. EMBO Mol. Med.
Curti, B.D., Faries, M.B., 2021. Recent Advances in the Treatment of Melanoma. N. Engl. J. Med. 384, 2229-2240.
De Velasco, G., 2017. Comprehensive Meta-analysis of Key Immune-Related Adverse Events from CTLA-4 and PD-1/PD-L1 Inhibitors in Cancer Patients. Cancer Immunol. Res. 5, 312-318.
Delyon, J., Lebbe, C., Dumaz, N., 2020. Targeted therapies in melanoma beyond BRAF: targeting NRAS-mutated and KIT-mutated melanoma. Curr. Opin. Oncol. 32, 79-84.
Dickson, P.V., Gershenwald, J.E., 2011. Staging and Prognosis of Cutaneous Melanoma. Surg. Oncol. Clin. N. Am. 20, 1-17.
Donati, B., Lorenzini, E., Ciarrocchi, A., 2018. BRD4 and Cancer: going beyond transcriptional regulation. Mol. Cancer 17, 164.
Fontanals-Cirera, B., 2017. Harnessing BET Inhibitor Sensitivity Reveals AMIGO2 as a Melanoma Survival Gene. Mol. Cell 68, 731-744.e9.
Guo, W., Wang, H., Li, C., 2021. Signal pathways of melanoma and targeted therapy. Signal Transduct. Target. Ther. 6, 424.
Hoek, K.S., Goding, C.R., 2010. Cancer stem cells versus phenotype-switching in melanoma. Pigment Cell Melanoma Res 23, 746-59.
Hogg, S.J., Beavis, P.A., Dawson, M.A., Johnstone, R.W., 2020. Targeting the epigenetic regulation of antitumour immunity. Nat. Rev. Drug Discov. 19, 776-800.
Huang, A.C., Zappasodi, R., 2022. A decade of checkpoint blockade immunotherapy in melanoma: understanding the molecular basis for immune sensitivity and resistance. Nat. Immunol. 23, 660-670.
Jenkins, R.W., Fisher, D.E., 2021. Treatment of Advanced Melanoma in 2020 and Beyond. J. Invest. Dermatol. 141, 23-31.
Jerby-Amon, L., 2018. A Cancer Cell Program Promotes T Cell Exclusion and Resistance to Checkpoint Blockade. Cell 175, 984-+.
Karras, P., 2022. A cellular hierarchy in melanoma uncouples growth and metastasis. Nature 610, 190-198.
Kozar, I., Margue, C., Rothengatter, S., Haan, C., Kreis, S., 2019. Many ways to resistance: How melanoma cells evade targeted therapies. Biochim. Biophys. Acta BBA - Rev. Cancer 1871, 313-322.
Kwiatkowski, N., 2014. Targeting transcription regulation in cancer with a covalent CDK7 inhibitor. Nature 511, 616-20.
Laham-Karam, N., 2018. Cutaneous melanoma: From pathogenesis to therapy (Review). Int. J. Oncol.
Love, M.I., Huber, W., Anders, S., 2014. Moderated estimation of fold change and dispersion for RNA-seq data with DESeq2. Genome Biol. 15, 1-21.
Luke, J.J., Flaherty, K.T., Ribas, A., Long, G.V., 2017. Targeted agents and immunotherapies: optimizing outcomes in melanoma. Nat. Rev. Clin. Oncol. 14, 463-482.
Luskin, M.R., Murakami, M.A., Manalis, S.R., Weinstock, D.M., 2018. Targeting minimal residual disease: a path to cure? Nat. Rev. Cancer 18, 255-263.
Marin-Bejar, O., 2021. Evolutionary predictability of genetic versus nongenetic resistance to anticancer drugs in melanoma. Cancer Cell 39, 1135-1149.e8.
Mehta, A., 2018. Immunotherapy Resistance by Inflammation-Induced Dedifferentiation. Cancer Discov. 8, 935-943.
Newell, F., 2022. Comparative Genomics Provides Etiologic and Biological Insight into Melanoma Subtypes. Cancer Discov. 12, 2856-2879.
Radke, J., 2022. Decoding molecular programs in melanoma brain metastases. Nat. Commun. 13, 7304.
Rambow, F., Marine, J.C., Goding, C.R., 2019. Melanoma plasticity and phenotypic diversity: therapeutic barriers and opportunities. Genes Dev 33, 1295-1318.
Rambow, F., 2018. Toward Minimal Residual Disease-Directed Therapy in Melanoma. Cell 174, 843.
Rebecca, V.W., Herlyn, M., 2020. Nongenetic Mechanisms of Drug Resistance in Melanoma. Annu. Rev. Cancer Biol. 4, 315-330.
Ribas, A., 2019. Five-Year Outcomes with Dabrafenib plus Trametinib in Metastatic Melanoma. N. Engl. J. Med. 381, 626-636.
Rubanov, A., Berico, P., Hernando, E., 2022. Epigenetic Mechanisms Underlying Melanoma Resistance to Immune and Targeted Therapies. Cancers 14, 5858.
Saginala, K., 2021. Epidemiology of Melanoma. Med. Sci. 9, 63.
Schadendorf, D., 2018. Melanoma. The Lancet 392, 971-984.
Sengupta, S., George, R.E., 2017. Super-Enhancer-Driven Transcriptional Dependencies in Cancer. Trends Cancer 3, 269-281.
Shain, A.H., Bastian, B.C., 2016. From melanocytes to melanomas. Nat Rev Cancer 16, 345-58.
Sung, H., Ferlay, 2021. Global Cancer Statistics 2020: GLOBOCAN Estimates of Incidence and Mortality Worldwide for 36 Cancers in 185 Countries. CA. Cancer J. Clin. 71, 209-249.
Switzer, B., 2022. Managing Metastatic Melanoma in 2022: A Clinical Review. JCO Oncol. Pract. 18, 335-351.
Tirosh, I., 2016. Dissecting the multicellular ecosystem of metastatic melanoma by single-cell RNA-seq. Science 352, 189-96.
Tsoi, J., 2018. Multi-stage Differentiation Defines Melanoma Subtypes with Differential Vulnerability to Drug-Induced Iron-Dependent Oxidative Stress. Cancer Cell 33, 890-904.e5.
Vervoort, S.J., 2022. Targeting transcription cycles in cancer. Nat. Rev. Cancer 22, 5-24.
Widmer, D.S., 2012. Systematic classification of melanoma cells by phenotype-specific gene expression mapping. Pigment Cell Melanoma Res 25, 343-53.
Wouters, J., 2020. Robust gene expression programs underlie recurrent cell states and phenotype switching in melanoma. Nat Cell Biol 22, 986-998.
Zanconato, F., 2018. Transcriptional addiction in cancer cells is mediated by YAP/TAZ through BRD4. Nat. Med. 24, 1599-161

### Clauses:

1. Compound IA 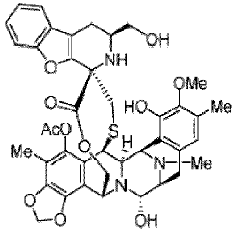 or a pharmaceutically acceptable salt or ester thereof, for use in treatment of skin cancer.
2. The compound for use according to clause 1, wherein the skin cancer is melanoma.
3. The compound for use according to clause 2, wherein the melanoma is invasive/undifferentiated.
4.The compound for use according to clause 2, wherein the melanoma is proliferative/differentiated.
5. The compound for use according to clause 2, wherein the melanoma is invasive/undifferentiated and proliferative/differentiated.
6. The compound for use according to any preceding clause, wherein the melanoma is unresectable or metastatic BRAF or RAS, or Triple WT mutated melanoma.
7. The compound for use according to any preceding clause, wherein the melanoma is selected from cutaneous melanoma, mucosal or acral melanoma.
8. The compound for use according to any preceding clause, wherein the melanoma is resistant to inhibitor(s) of MAP kinase signaling pathway.
9. The compound for use according to clause 8, wherein the inhibitors of MAP kinase signaling pathway are selected from BRAF or MEK kinase inhibitors or a combination thereof.
10. The compound for use according clause 9, wherein the BRAF or MEK kinase inhibitors are selected from vemurafenib, dabrafenib, encorafenib, trametinib, cobimetinib or binimetinib or a combination thereof.
11. The compound for use according clause 10, wherein the BRAF or MEK kinase inhibitors are vemurafenib, dabrafenib, or trametinib or a combination thereof.
12. The compound for use according clause 9, wherein the melanoma is resistant to BRAF and MEK kinase inhibitors, including dabrafenib plus trametinib, vemurafenib plus cobimetinib, and encorafenib plus binimetinib.
13. The compound for use according to clauses 8 to 12, wherein the melanoma is resistant to vemurafenib.
14. A pharmaceutical composition or dosage form comprising a compound as defined in clause 1, for use in the treatment of skin cancer as defined in any preceding clause.
15. A pharmaceutical package comprising a compound as defined in clause 1, together with instructions for its use in the treatment of skin cancer as defined in any preceding clause.
16. A method of inhibiting cancer cell growth, comprising contacting cancer cells with a therapeutically effective amount of compound as defined in clause 1, wherein said cancer cells are skin cancer cells.
17. PM14 compound 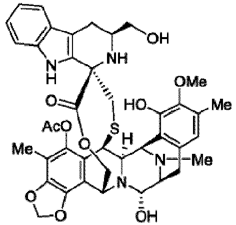 or a pharmaceutically acceptable salt or ester thereof, for use in treatment of invasive/undifferentiated and/or proliferative/differentiated melanoma.
18. The compound for use according to clause 17, wherein the melanoma is invasive/undifferentiated.
19. The compound for use according to clause 17, wherein the melanoma is proliferative/differentiated.
20. The compound for use according to clause 17, wherein the melanoma is invasive/undifferentiated and proliferative/differentiated.
21. The compound for use according to any one of clauses 17 to 20, wherein the melanoma is unresectable or metastatic BRAF or RAS, or Triple WT mutated melanoma.
22. The compound for use according to any one of clauses 17 to 21, wherein the melanoma is selected from cutaneous melanoma, mucosal or acral melanoma.
23. The compound for use according to any one of clauses 17 to 22, wherein the melanoma is resistant to inhibitor(s) of MAP kinase signaling pathway.
24. The compound for use according to clause 23, wherein the inhibitors of MAP kinase signaling pathway are selected from BRAF or MEK kinase inhibitors or a combination thereof.
25. The compound for use according clause 24, wherein the BRAF or MEK kinase inhibitors are selected from vemurafenib, dabrafenib, encorafenib, trametinib, cobimetinib or binimetinib or a combination thereof.
26. The compound for use according clause 25, wherein the BRAF or MEK kinase inhibitors are vemurafenib, dabrafenib, or trametinib or a combination thereof.
27. The compound for use according clause 24, wherein the melanoma is resistant to BRAF and MEK kinase inhibitors, including dabrafenib plus trametinib, vemurafenib plus cobimetinib, and encorafenib plus binimetinib.
28. The compound for use according to any one of clauses 17 to 27, wherein the melanoma is resistant to vemurafenib.
29. A pharmaceutical composition or dosage form comprising a compound as defined in clause 17, for use in the treatment of skin cancer as defined in any one of clauses 17 to 28.
30. A pharmaceutical package comprising a compound as defined in clause 17, together with instructions for its use in the treatment of skin cancer as defined in any one of clauses 17 to 28.
31. A method of inhibiting cancer cell growth, comprising contacting cancer cells with a therapeutically effective amount of compound as defined in clause 17, wherein said cancer cells are melanoma cancer cells.
32. Lurbinectedin 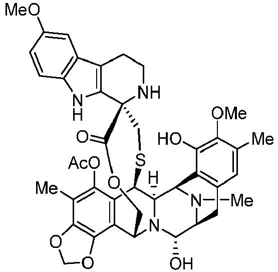 or a pharmaceutically acceptable salt or ester thereof, for use in treatment of invasive/undifferentiated and/or proliferative/differentiated melanoma.
33. The compound for use according to clause 32, wherein the melanoma is invasive/undifferentiated.
34. The compound for use according to clause 32, wherein the melanoma is proliferative/differentiated.
35. The compound for use according to clause 32, wherein the melanoma is invasive/undifferentiated and proliferative/differentiated.
36. The compound for use according to any one of clauses 32 to 35, wherein the melanoma is unresectable or metastatic BRAF or RAS, or Triple WT mutated melanoma.
37. The compound for use according to any one of clauses 32 to 36, wherein the melanoma is selected from cutaneous melanoma, mucosal or acral melanoma.
38. The compound for use according to any one of clauses 32 to 37, wherein the melanoma is resistant to inhibitor(s) of MAP kinase signaling pathway.
39. The compound for use according to clause 38, wherein the inhibitors of MAP kinase signaling pathway are selected from BRAF or MEK kinase inhibitors or a combination thereof.
40. The compound for use according clause 39, wherein the BRAF or MEK kinase inhibitors are selected from vemurafenib, dabrafenib, encorafenib, trametinib, cobimetinib or binimetinib or a combination thereof.
41. The compound for use according clause 40, wherein the BRAF or MEK kinase inhibitors are vemurafenib, dabrafenib, or trametinib or a combination thereof.
42. The compound for use according clause 39, wherein the melanoma is resistant to BRAF and MEK kinase inhibitors, including dabrafenib plus trametinib, vemurafenib plus cobimetinib, and encorafenib plus binimetinib.
43. The compound for use according to any one of clauses 32 to 42, wherein the melanoma is resistant to vemurafenib.
44. A pharmaceutical composition or dosage form comprising a compound as defined in clause 32, for use in the treatment of skin cancer as defined in any one of clauses 32 to 43.
45. A pharmaceutical package comprising a compound as defined in clause 32, together with instructions for its use in the treatment of skin cancer as defined in any one of clauses 32 to 43.
46. A method of inhibiting cancer cell growth, comprising contacting cancer cells with a therapeutically effective amount of compound as defined in clause 32, wherein said cancer cells are melanoma cancer cells.

## Claims

1. A compound selected from Compound IA, PM14 and lurbinectedin 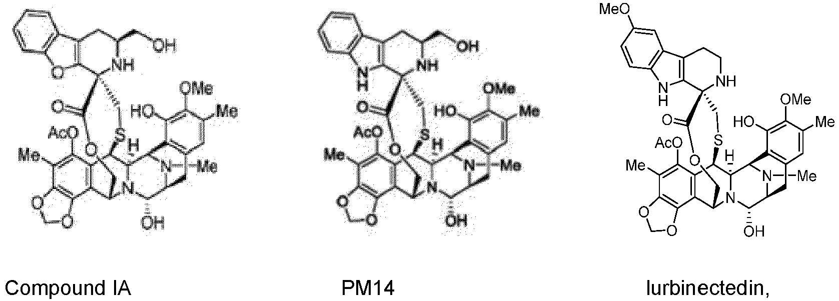 or pharmaceutically acceptable salts or esters thereof, for use in treatment of cancer, wherein the cancer is invasive/undifferentiated and/or proliferative/differentiated melanoma.

2. A compound which is Compound IA 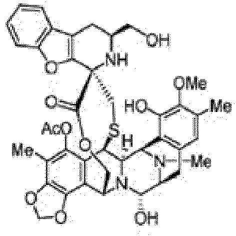 or a pharmaceutically acceptable salt or ester thereof, for use in treatment of cancer, wherein the cancer is skin cancer.

3. The compound for use according to claim 2, wherein the skin cancer is melanoma.

4. The compound for use according to any preceding claim, wherein the cancer is invasive/undifferentiated melanoma.

5. The compound for use according to any preceding claim, wherein the cancer is proliferative/differentiated melanoma.

6. The compound for use according to any preceding claim, wherein the cancer comprises invasive/undifferentiated and proliferative/differentiated melanoma.

7. The compound for use according to any preceding claim, wherein the cancer is unresectable or metastatic BRAF or RAS, or Triple WT mutated melanoma.

8. The compound for use according to any preceding claim, wherein the cancer is selected from cutaneous melanoma, mucosal melanoma or acral melanoma.

9. The compound for use according to any preceding claim, wherein the cancer is melanoma and is resistant to inhibitor(s) of MAP kinase signaling pathway.

10. The compound for use according to claim 9, wherein the inhibitors of MAP kinase signaling pathway are selected from BRAF or MEK kinase inhibitors or a combination thereof.

11. The compound for use according to claim 10, wherein the BRAF or MEK kinase inhibitors are selected from vemurafenib, dabrafenib, encorafenib, trametinib, cobimetinib or binimetinib or a combination thereof.

12. The compound for use according to claim 11, wherein the BRAF or MEK kinase inhibitors are vemurafenib, dabrafenib, or trametinib or a combination thereof.

13. The compound for use according to claim 10, wherein the cancer is melanoma which is resistant to BRAF and MEK kinase inhibitors.

14. The compound for use according to claim 13, wherein the cancer is melanoma which is resistant to dabrafenib plus trametinib and/or vemurafenib plus cobimetinib and/or encorafenib plus binimetinib.

15. The compound for use according to claims 9 to 14, wherein the cancer is melanoma which is resistant to vemurafenib.
